(19) Europäisches Patentamt
European Patent Office
Office européen des brevets

(11) **EP 3 278 839 A1**

(12) **EUROPEAN PATENT APPLICATION**

(43) Date of publication:
**07.02.2018 Bulletin 2018/06**

(51) Int Cl.:
**A61N 7/00** *(2006.01)* **A61B 18/00** *(2006.01)*
**A61B 17/00** *(2006.01)* **A61B 90/00** *(2016.01)*

(21) Application number: **17183821.2**

(22) Date of filing: **28.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.08.2016 US 201615229804**

(71) Applicant: **Axiosonic, LLC**
**Effingham, IL 62401 (US)**

(72) Inventors:
• **Bonutti, Peter M.**
**Manalapan, Florida 33462 (US)**
• **Beyers, Justin E.**
**Effingham, Illinois 62401 (US)**
• **Bierman, Tonya M.**
**Dieterich, Illinois 62424 (US)**

(74) Representative: **Hocking, Adrian Niall et al**
**Albright IP Limited**
**County House**
**Bayshill Road**
**Cheltenham, Glos. GL50 3BA (GB)**

(54) **SYSTEMS USING ULTRASOUND FOR TREATMENT**

(57) A device (100) for treating infection within a subject includes an ultrasound transducer (310) for applying ultrasound to a treatment site of the subject.

## FIG. 3

**Description**

BACKGROUND

The field of the disclosure relates generally to systems and methods for using ultrasound for treatment in the healthcare field.

Generally, it has been shown that some infections are resistant to conventional treatments, such as antibiotics alone. For example, biofilm and Methicillin-resistant *Staphylococcus aureus* are resistant to antibiotic treatment alone. It is also known that some treatments in the healthcare field need improvements.

SUMMARY

In one aspect, a device for treating infection within a subject comprises an ultrasound transducer for applying ultrasound to a treatment site of the subject.

In a second aspect in accordance with the present invention, there is provided an ultrasound treatment device comprising: a hand-held treatment applicator configured to acoustically interface with a treatment site of a subject; an ultrasound transducer disposed in the applicator, the ultrasound transducer configured to generate ultrasound energy, wherein the ultrasound transducer is coupled to the applicator to deliver the generated ultrasound energy to the treatment site when the hand-held treatment applicator is acoustically interfaced with the treatment site; a vibrator mounted on the applicator, the vibrator configured to generate vibratory energy, wherein the vibrator is coupled to the applicator to deliver the generated vibratory energy to the hand of the user to provide haptic feedback to the user; and a control circuit electrically connected to the ultrasound transducer and the vibrator, the control circuit configured to selectively supply electrical energy to the ultrasound transducer to generate ultrasound energy and to selectively supply electrical energy to the vibrator to provide haptic feedback to the user.

Preferable options relating to the second aspect of the invention are set forth in claims 2 to 15.

In a third aspect not in accordance with the present invention, there is provided a method of treating a subject comprising: providing an ultrasound treatment device including: a hand-held treatment applicator configured to acoustically interface with a treatment site of a subject, an ultrasound transducer disposed in the applicator, the ultrasound transducer configured to generate ultrasound energy, wherein the ultrasound transducer is coupled to the applicator to deliver the generated ultrasound energy to the treatment site when the hand-held treatment applicator is acoustically interfaced with the treatment site, a vibrator disposed in the applicator, the vibrator configured to generate vibratory energy, wherein the vibrator is coupled to the applicator to deliver the generated vibratory energy to the hand of the user to provide haptic feedback to the user, and a control circuit electrically connected to the ultrasound transducer and the vibrator, the control circuit configured to selectively supply electrical energy to the ultrasound transducer to generate ultrasound energy and selectively supply electrical energy to the vibrator to provide haptic feedback to the user; acoustically coupling the hand-held treatment applicator of the ultrasound treatment device to the treatment site of the subject; transmitting ultrasonic energy generated by the ultrasound transducer to the treatment site of the subject after said acoustically coupling; and delivering vibratory energy generated by the vibrator to the hand of the user simultaneously with said acoustically coupling the hand-held treatment applicator.

The ultrasound treatment device may further include coupling sensor operatively connected to the control circuit, wherein the coupling sensor may be configured to generate a coupling signal indicative of the amount of ultrasonic energy generated by the ultrasound transducer being transmitted outward from the hand-held treatment applicator, the method may further comprise of: generating, after said transmitting ultrasonic energy and before said delivering vibratory energy, the coupling signal via the coupling sensor; and receiving, after said transmitting ultrasonic energy and before said delivering vibratory energy, the coupling signal, the generated coupling signal via the control circuit.

The method may further comprise of determining, after said transmitting ultrasonic energy and before said delivering vibratory energy, the generated coupling signal is indicative of the amount of ultrasonic energy generated by the ultrasound transducer being transmitted outward from the hand-held treatment applicator being greater than or greater than or equal to a predetermined threshold amount of ultrasonic energy via the control circuit.

The control circuit may be configured to selectively adjust the electrical energy supplied to the vibrator to adjust the vibratory energy generated by the vibrator, the method may further comprise of: adjusting the electrical energy supplied to the vibrator to adjust the vibratory energy generated by the vibrator.

The said adjusting the electrical energy supplied to the vibrator may comprise at least one of increasing and decreasing the amount of electrical energy supplied to the vibrator, and/or adjusting a waveform of the electrical energy supplied to the vibrator.

The features, functions, and advantages that have been discussed can be achieved independently in various embodiments or may be combined in yet other embodiments, further details of which can be seen with reference to the following description and drawings.

BRIEF DESCRIPTION OF THE DRAWINGS

FIG. 1 is a perspective view of an exemplary high frequency ultrasound (HFUS) device for affecting bacteria, biofilm, and/or infection within the body.

FIG. 2 is a block diagram of circuitry positioned in the device housing shown in FIG. 1.

FIG. 3 is a cross section of the treatment applicator shown in FIG. 1.

FIG. 4 is a perspective view of an alternative HFUS device having the components of the device shown in FIG. 1.

FIG. 5 is schematic diagram of potential treatment regions of a patient that may be used with the device shown in FIG. 1.

FIG. 6 is a graphical representation of the results of Test 1 using the device shown in FIG. 1.

FIG. 7 displays microscope images of the results of Test 2 using the device shown in FIG. 1.

FIG. 8 displays microscope images of the results of Test 3 using the device shown in FIG. 1.

FIG. 9 is an exemplary flowchart of a method for use with the device shown in FIG. 1.

FIG. 10 is an exemplary flowchart of a method for use with the device shown in FIG. 1.

FIG. 11 is an exemplary drive waveform that may be used with the applicator shown in FIG. 1.

FIG. 12 is an exemplary drive section that may be used with the applicator shown in FIG. 1.

FIG. 13 is an alternative drive section that may be used with the applicator shown in FIG. 1.

FIG. 14 is an alternative drive section that may be used with the applicator shown in FIG. 1.

FIG. 15 is an alternative drive section that may be used with the applicator shown in FIG. 1.

FIG. 16 is a perspective view of an alternative HFUS device having the components of the device shown in FIG. 1.

FIG. 17 is a perspective view of the HFUS device of FIG. 1 with a display showing a graphical representation of a human body.

FIG. 18 is a perspective view of the HFUS device of FIG. 1 with a display showing a graphical representation of a human knee.

FIG. 19 is a schematic showing the HFUS device of FIG. 1 treating a human knee.

FIG. 20 is a schematic showing the HFUS device of FIG. 1 treating a stent in a human body.

FIG. 21 is a schematic showing the HFUS device of FIG. 1 treating a screw in a human body.

FIG. 22 is a schematic showing the HFUS device of FIG. 1 treating mesh in a human body.

FIG. 23 is a cross section of another embodiment of a treatment applicator similar to the treatment applicator of FIG. 1.

FIG. 24 is a perspective of another embodiment of a high frequency ultrasound (HFUS) device for affecting bacteria, biofilm, and/or infection within the body, similar to the embodiment illustrated in FIG. 1.

FIG. 25 is a front elevational view of FIG. 24.

FIG. 26 is a front elevation of a treatment applicator mask positioned on the head of a subject with a control circuit and ultrasound transducers illustrated schematically.

FIG. 27 is an elevation of a treatment system including a treatment applicator and a smartphone connected to the treatment applicator by a wired connection.

FIG. 28 is an elevation of the treatment applicator of FIG. 27 illustrating components of a control circuit thereof in a schematic block diagram superimposed on the treatment applicator.

FIG. 29 is an elevation of a treatment applicator configured for treatment of otitis media with a control circuit and a power supply thereof illustrated schematically.

FIG. 30 is an elevation of a nozzle of the treatment applicator of Fig. 29.

FIG. 31 is an elevation of a treatment applicator configured for applying a combination treatment of topical therapeutic agent and therapeutic ultrasound.

FIG. 32 is an elevation another embodiment of a treatment applicator configured for applying a combination treatment of topical therapeutic agent and therapeutic ultrasound wherein the therapeutic agent is provided by a disposable applicator pad.

FIG. 33 is a perspective of a therapeutic bath and a foot of a subject received in the bath.

FIG. 34 is an elevation of another embodiment of a treatment applicator mask with a control circuit and ultrasound transducers illustrated schematically.

FIG. 35 is a schematic elevation of a treatment applicator patch.

FIG. 36 is a schematic elevation of a treatment applicator stocking.

FIG. 37 is a perspective of a treatment applicator bridle positioned on the head of a horse.

FIG. 38 is a perspective of a treatment applicator patch positioned on the utter of a cow.

FIG. 39 is an elevation of a treatment applicator patch positioned over a portion of a trunk of a tree.

FIG. 40 is an exemplary flowchart of a method for determining the thermal index of a treatment site.

FIG. 41 is a diagram of the growth rate versus temperature for five environmental classes of procaryotes.

Corresponding parts are given corresponding reference numbers throughout the drawings.

DETAILED DESCRIPTION

In one embodiment, the systems and methods described herein enable treatment of infection of a living subject (i.e., a human or other animal) using high frequency ultrasound (HFUS). As used herein, the term "infection" refers to an invasion of the living subject by an infectious agent, regardless of whether the infectious agent causes a disease. Non-limiting examples of infectious agents causing infection include bacteria, viruses, fungi, parasites, and prions. The infectious agent(s) causing the infection may exist in the living subject in a planktonic state or as biofilm. As used herein, infectious agents causing the infection are in a planktonic state (i.e., a planktonic infection) if the infectious agents are free-floating within the subject, and the infectious agents are in a biofilm (i.e., a biofilm infection) if the infectious agents are microorganisms adhered to each other on a surface within the subject and are enclosed by a self-produced matrix of a secreted extracellular polymeric substance. The biofilm extracellular polymeric substance excreted by the biofilm infection may comprise polysaccharides (e.g., exopolysaccharides), proteins, DNA, lipids and humic substances. Examples of infectious agents forming biofilms described herein include, but are not limited to, bacteria, archaea, protozoa, fungi, and algae.

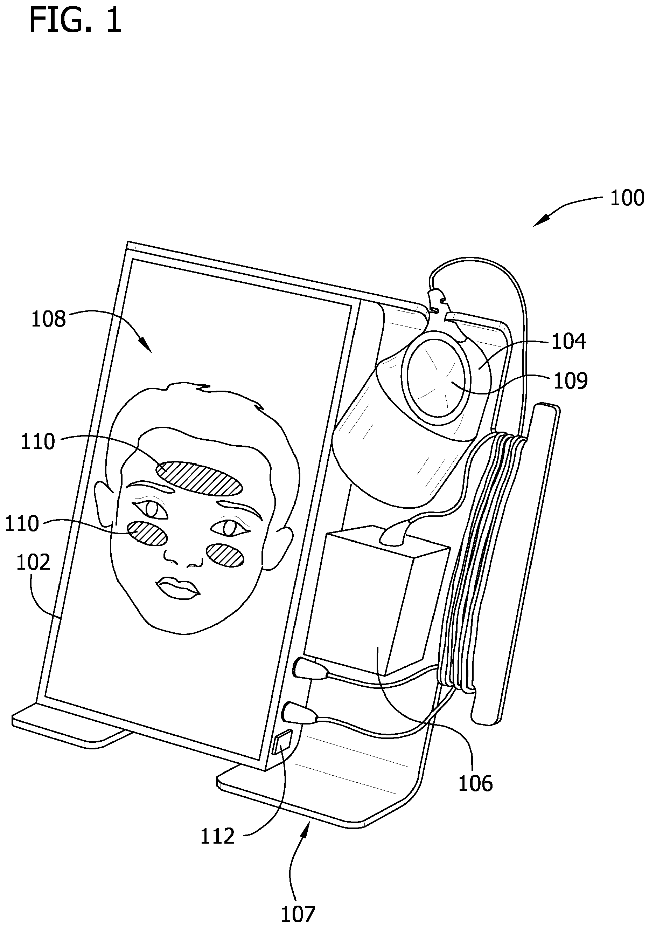

FIG. 1 is a perspective view of an exemplary high frequency ultrasound (HFUS) device, generally indicated at 100, for treating infection of a living subject. In general, the device is configured to deliver ultrasonic energy (e.g., high frequency ultrasonic energy) to a site of infection of the living subject to treat (i.e., combat, ameliorate, inhibit, and/or prevent) the infection. Device 100 comprises a device housing 102, a treatment applicator 104 including an ultrasonic transducer 310, and a control circuit 200 contained within the housing for controlling the output of the ultrasonic transducer. In one embodiment, device 100 is powered by an AC power adapter 106 (e.g., an external or internal AC power adapter) configured to receive AC power from a power source (e.g., mains power) and convert the AC power to DC power used by device 100. In the illustrated embodiment, the HFUS device 100 also includes a DC power source within the housing 102. As a non-limiting example, DC power source may be a battery, including but not limited to, a rechargeable lithium-ion battery (e.g., battery and charger circuit 202). The HFUS device 100 may be powered in other ways without departing from the scope of the present invention.

In one embodiment, applicator 104 and/or housing 102 are configured to be hand-held and portable such that a user can utilize applicator 104 and/or housing 102 with one hand. In some embodiments, applicator 104 and/or housing 102 are configured to have an ergonomic design when held by a user. For example, in the illustrated embodiment applicator 104 includes a recess 109 that contours to one or two fingers that aid in stabilization of applicator 104. Recess 109 also enables a user to hold applicator 104 with a pinch grip for ease of use. Housing 102 and applicator 104 are storable on a base 107 (e.g., a stand). Housing 102 and/or applicator 104 may be removably coupled to base 107, such as by magnets (not shown)

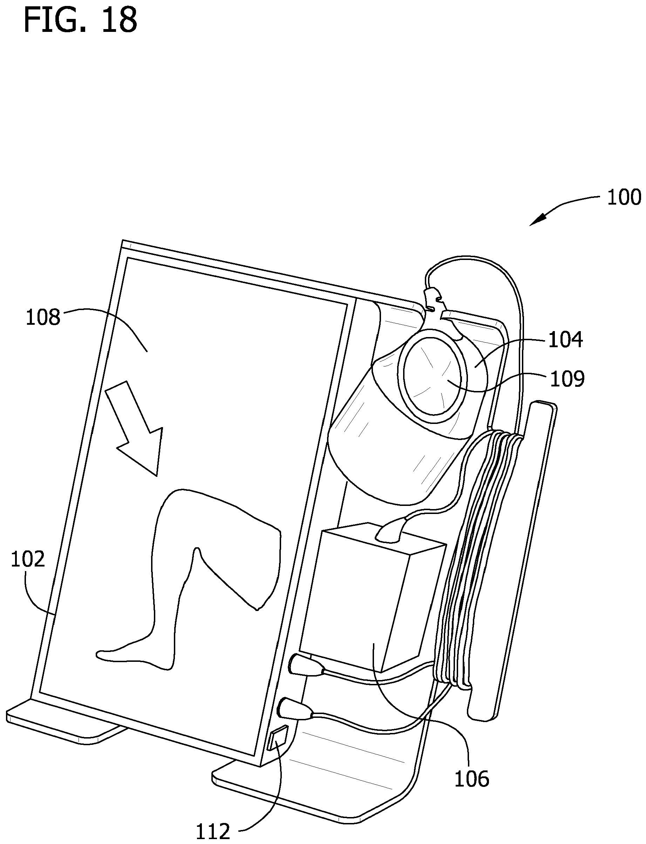

A user interface 108 is provided on housing 102 to allow communication between the user and device 100, in particular between the user and the control circuit 200. User interface 108 has a presentation function configured to present information, such as treatment information and/or execution events, to a user. For example, user interface 108 may include a display device, as illustrated, for presenting information to a user. The display device may include a cathode ray tube (CRT), a liquid crystal display (LCD), LED, an organic LED (OLED) display, a vacuum fluorescent display (VFD), and/or an "electronic ink" display. In some embodiments, user interface 108 may include one or more display devices. In the illustrated embodiment, user interface 108 displays the intended application area and/or configuration of device 100 for treating infection of a user. For example, as illustrated in FIG. 1, user interface 108 comprises a display generating a graphical representation of a human face to which treatment of infection using device 100 is to be applied. In other examples, device 100 may be configured to generate a graphic representation of another portion(s) of a human body or the entire human body on the display. For example, as shown in FIG. 18 a graphical representation of a knee or other joint of the body may be generated on the display to indicate the desired treatment site. Data may be stored in a remote database, such as cloud storage.

In the exemplary embodiment, user interface 108 also has an input function to allow a user to communicate with device 100, in particular control circuit 200. As an example, to allow a user to communicate with device 100, user interface 108 may include keys, a pointing device, a mouse, a stylus, a membrane switch, a touch sensitive panel (e.g., a touch pad or a touch screen), a gyroscope, an accelerometer, a position detector, and/or an audio user input interface. In the illustrated embodiment, user interface 108 comprises a touch screen having both presentation and input functions. In one example, user interface 108 may be configured to receive an input from the user as to a desired treatment area and/or treatment protocol. In the illustrated embodiment user interface 108 (i.e., touch screen) may be configured to allow the user to select a body portion for treatment and/or a specific area of a body portion for treatment. For example, as illustrated user interface 108 allows a user to select a sinus area for treatment by touching the desired sinus area on the display. This selection is communicated to control circuit 200, as explained in more detail below.

In the exemplary embodiment, a communication interface 112 coupled to control circuit 200 is provided on housing 102. Communication interface 112 communicates with control circuit 200 to allow transfer of treatment and/or session information stored by device 100. To communicate with control circuit 200, communication interface 112 may include, for example, a wired network adapter, a wireless network adapter, and/or a mobile telecommunications adapter. In some embodiments, communication interface 112 is a direct link interface for linking two computing devices, the direct link interface including, but not being limited to, a serial port, a firewire port, a USB port, and an Ethernet port. In one embodiment, communication interface 108 includes a Bluetooth adapter capable of communication with a Bluetooth receiver positioned in a separate computing device (e.g., tablet, pc, smartphone, and smartwatch). In some embodiments, communication interface 112 receives information such as executable instructions and/or other data that can be stored and/or executed by control circuit 200.

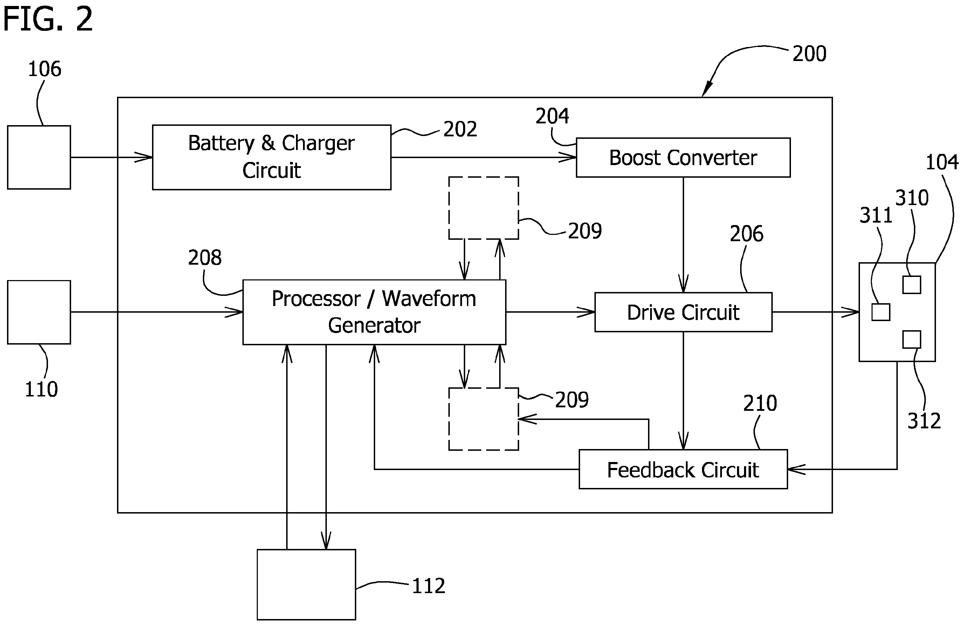

FIG. 2 is a block diagram of device 100 illustrated in FIG. 1. As shown in FIG. 2, power source 106 is electrically connected to a battery and charger circuit 202. Electrical power (e.g., DC current) is delivered from power source 106 (e.g., AC adapter) to battery and charger circuit 202. Battery and charger circuit 202 is electrically connected to boost converter 204. Battery and charger circuit 202 transmits electrical power (e.g., DC power) to boost converter 204. Boost converter 204 is electrically connected to drive circuit 206. Boost converter 204 outputs DC power to drive circuit 206 having a DC voltage that is greater than the DC voltage of the power received from battery and charger circuit 202.

A processor 208 of control circuit 200 is electrically connected to user interface 110, communication interface 112, and drive circuit 206. In the illustrated embodiment, processor 208 is configured to execute instructions provided on non-transitory computer readable medium, such as memory device 209. Instructions provided on memory device 209 include instructions for operating device 100 to treat infection of a subject using applicator 104, as explained below. Processor 208 communicates with user interface 110 to receive commands from a user and output information to the user, as described below. In the illustrated embodiment, processor 208 operates as a waveform generator, whereby an electrical signal is delivered to drive circuit 206 in accordance with the desired frequency output of ultrasound transducer 310. In particular, drive circuit 206 is configured to receive DC power from boost converter 204 and a waveform electrical signal from processor 208. The drive circuit 206 delivers an AC drive signal to transducer 310 of applicator 104 based on the waveform electrical signal and the DC power received from the boost converter 204. Transducer 310 outputs the desired HFUS energy (i.e., having a desired frequency and intensity) in accordance with the received drive signal. The outputted HFUS energy is suitable for treating infection of the subject. The output of transducer 104 may be monitored by a feedback circuit 210 in communication with processor 208.

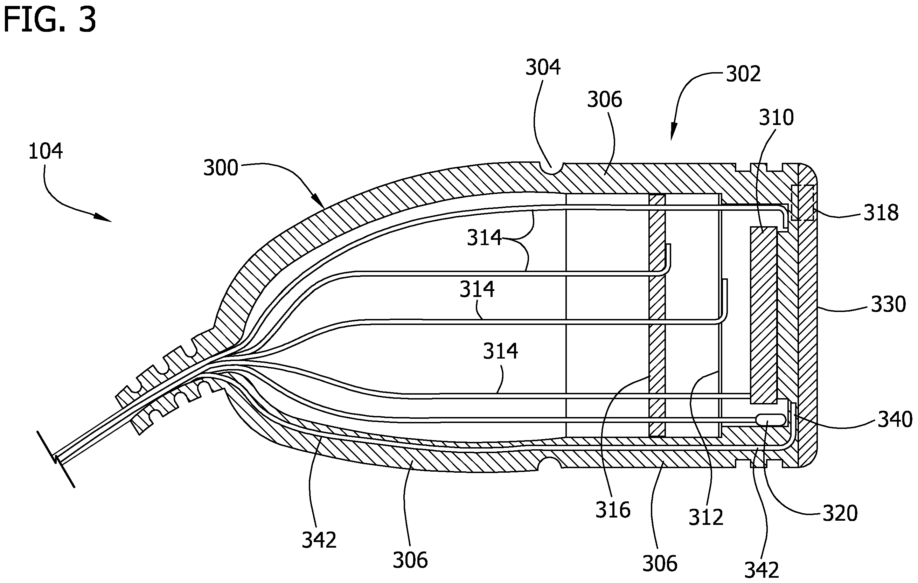

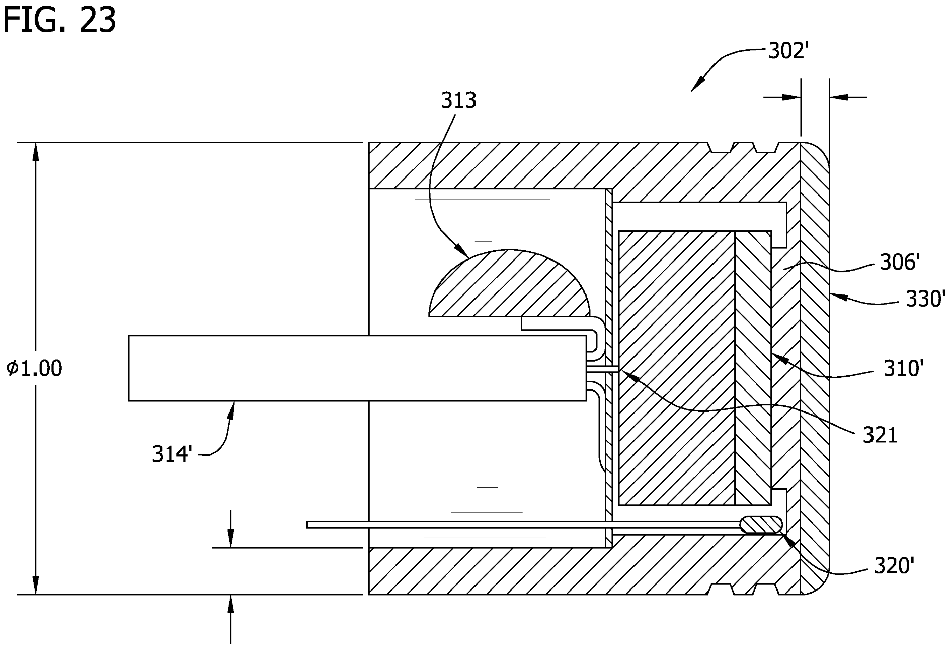

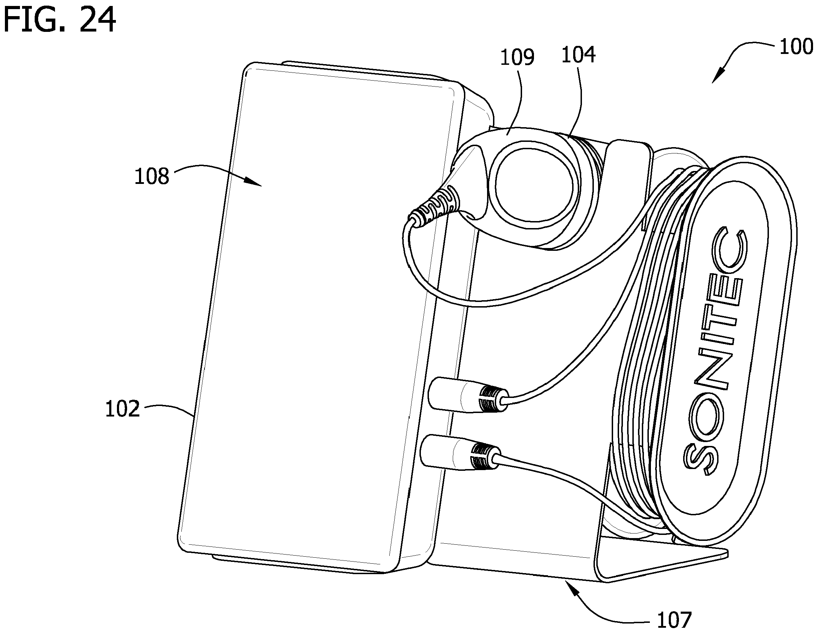

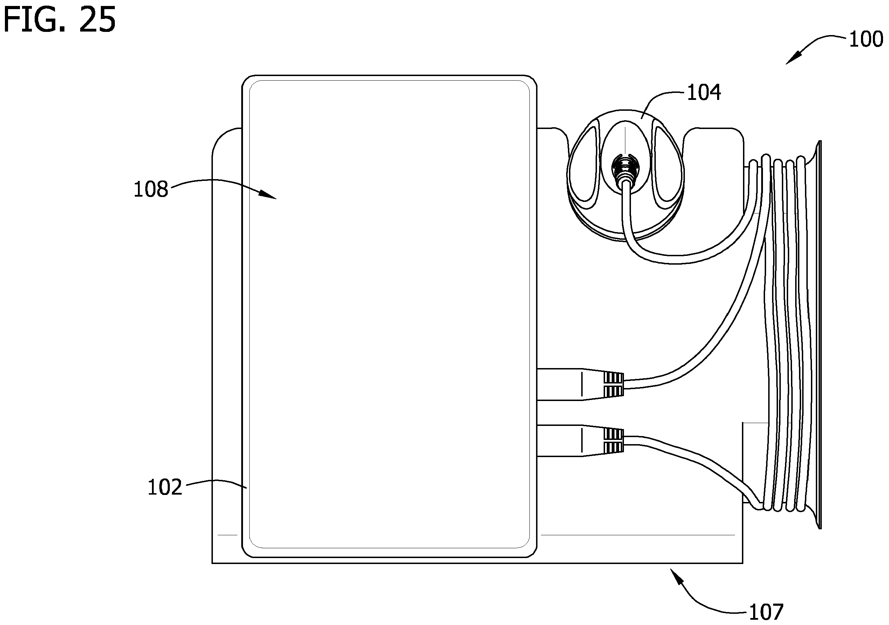

FIG. 3 is a side cut-away view of applicator 104 shown in FIG. 1. In the exemplary embodiment, applicator 104 includes shell 306 having a body portion, generally indicated at 300, and an applicator head portion, generally indicated at 302, configured to provide the HFUS energy to a treatment site in the body. A similar embodiment of a head portion 302' is shown in FIG. 23, with differences between the embodiments discussed below. In some embodiments, a retention groove 304 is formed on shell 306. Retention groove 304 is configured to enable applicator 104 be held by a system retention apparatus, such as a clip or stand provided on base 107. For example, as shown in FIGS. 24 and 25, in another embodiment the base 107 incudes a U-shaped cutout 305 in which the applicator 104 is retained. A bottom edge defining the U-shaped cutout 305 is received in the retention groove 304 of the applicator 104 to hold the applicator in the U-shaped cutout. In one embodiment, shell 306 of applicator 104 is fabricated from a polymer, including but not limited to, acrylonitrile butadiene styrene, polyether ether ketone, Polyoxymethylene. Alternatively, shell 306 can be fabricated from any material that facilitates transmitting energy (e.g. ultrasonic vibrations) from applicator 104 to a treatment site of the subject, including but not limited to, titanium, aluminum, and stainless steel. In another embodiment, body portion 300 of outer shell 306 may be fabricated from a polymer and head portion 302 may be fabricated from a metallic substance (e.g. titanium).

As described above, transducer 310 is configured to convert drive signal into ultrasonic vibratory energy that will be utilized to treat infection at a treatment site of the subject. In the exemplary embodiment, transducer 310 is configured to output ultrasound energy having a frequency selected to be above 20 kHz, such as from about 1 MHz to about 5 MHz, and an intensity from about 0.20 W/cm$^2$ to about 3 W/cm$^2$, such as from about 0.5 W/cm$^2$ to about 1 W/cm$^2$, in accordance with the drive signal received from drive circuit 206. Transducer 310 may comprise a piezoelectric crystal having a square shape or any other suitable shape, including but not limited to, round, circular, oval, and rectangular. In at least some embodiments, applicator 104 includes a plurality or array of transducers 310 for transmitting ultrasonic vibratory energy. In such embodiments, transducers 310 are arranged to focus ultrasound at a treatment site, with two or more transducers 310 outputting different frequencies such that the intersection of the ultrasound beams will create a different frequency at the desired treatment site. Alternatively, discrete transducers 310 are configured to provide different beams that are configured to affect particular portions (e.g. proximal, distal, etc.) of a treatment site. In some embodiments, discrete transducers 310 are configured to simultaneously provide multiple beams that are configured to treat different types of infections (e.g. MRSA infection, bacterial biofilm infection, and fungal biofilm infection).

In some embodiments, the frequency of the output of transducer 310 is 1 MHz. An output of ultrasonic vibratory energy of 1 MHz has a beneficial effect on pain and swelling. Additionally, it is believed the output of ultrasonic vibratory energy at a frequency of 1 MHz has an effect on infectious agents and/or biofilms by turning infectious agents, such as bacteria, and/or biofilm into planktonic state, causing delamination of biofilm, creating a physical disruption, and/or breakdown of polysaccharides present in biofilm matrix. Further, with respect to treating infection of a sinus cavity, the application of the high frequency ultrasound can have an effect on the viscosity of mucus in the sinus cavity which can enhance drainage.

In the exemplary embodiment (FIG. 1), applicator 104 includes a vibratory device 312, such as but not limited to a piezoelectric transducer or an eccentric vibrator motor, that provides tactile vibrations to the user. Such an embodiment enables a user to feel that applicator 104 is functioning and/or in a treatment mode. Vibratory device 312 may be configured to operate, and thereby vibrate, when the ultrasound transducer 310 is outputting the ultrasound signal during treatment. In one embodiment, vibratory device 312 may be powered by the drive signal from drive circuit 206, while the drive signal simultaneously powers ultrasound transducer 310. In the exemplary embodiment, ultrasonic transducer 310 and vibratory transducer 312 are configured to receive power simultaneously from electrical power supply 202. Alternately, transducers 310 and 312 can be configured to receive power individually. In the embodiment illustrated in FIG. 23, the head portion 302' includes a vibratory device 313 comprising an eccentric vibrator motor 313. Other types of vibratory devices do not depart from the scope of the present invention.

In the exemplary embodiment (FIG. 1), applicator 104 includes an imaging transducer 311 (e.g., a transceiver) for sending and receiving ultrasound suitable for imaging the treatment site. Processor 208 may be configured to process the ultrasound for imaging, as explained in more detail below.

In some embodiments, applicator 104 is configured to provide multiple treatment modalities to treat infection. In addition to providing ultrasound energy by operation of ultrasonic transducer 310, device 100 may be configured to apply additional energy, other than HFUS energy, to the treatment site. For example, applicator 104 may include additional treatment components 316 and/or 318. In one embodiment, treatment component 316 comprises a transducer configured to provide extracorporeal shockwaves to the treatment site and/or pulsed electromagnetic frequency (PEMF) energy to a treatment site. In one embodiment, component 318 is a conductor configured to provide electrical AC current in the radio frequency range or DC current. In yet another embodiment, the treatment component 316 may include a source of ultraviolet light for using in treating in conjunction with the ultrasonic transducer. The source of ultraviolet light may emit light in the C range from about 270 nanometers to approximately 320 nanometers.

To inhibit overheating of applicator 104, a temperature sensor 320 is coupled within applicator 104 to provide temperature feedback to processor 208. If the temperature sensed by temperature sensor 320 is greater than a threshold temperature, processor 208 may be configured to reduce intensity of the drive signal or discontinue treatment using device 100 until the temperature falls within an acceptable range. In the embodiment illustrated in FIG. 23, the head portion 302 includes a temperature sensor 320' and a heat sink 321 to reduce overheating of the applicator 104. The heat sink 321 is in thermal contact with the transducer 310' to transfer heat from the transducer to the heat sink to inhibit overheating of the shell 306'. The heat sink 321 may comprise any suitable thermally conductive material having a thermal conductivity greater than the shell 306', for example.

In the illustrated embodiment, a transmission component 330 is coupled to head portion 302 of applicator 104. Transmission component 330 is fabricated to enable transmission of ultrasound energy from applicator 104 into the body of a subject without a coupling gel. In one embodiment, transmission component 330 is an overmold coupled on head portion 302. In the exemplary embodiment transmission component 330 is fabricated from silicone. Alternatively, transmission component 330 can be fabricated from any material that enables the transmission of energy from applicator 104 to a treatment site within the body of the subject including, but not limited to, an ultra-high-molecular-weight polyethylene, a thermoplastic elastomer, and polytetrafluoroethylene. In some embodiments, transmission component 330 is a reservoir that includes an aperture for inserting and extracting material in the reservoir. In such an embodiment, gels and/or other substances capable of transmitting energy from applicator 104 to a treatment site within the body can be heated or cooled before inserting into the reservoir to provide heating or cooling to tissue that contacts transmission component 330. A specific drain or aspirate can be provided in addition to the vibratory circuit.

In one embodiment, head portion 302 includes at least one aperture 340 in a portion of head portion 302 that contacts the skin of the user (e.g. transmission component 330). In such an embodiment, a suction component 342 may be coupled to the at least one aperture 340 to provide suction pressure and create a partial vacuum at the skin of a user to aid in retaining applicator 104 against the skin of a user during a treatment session.

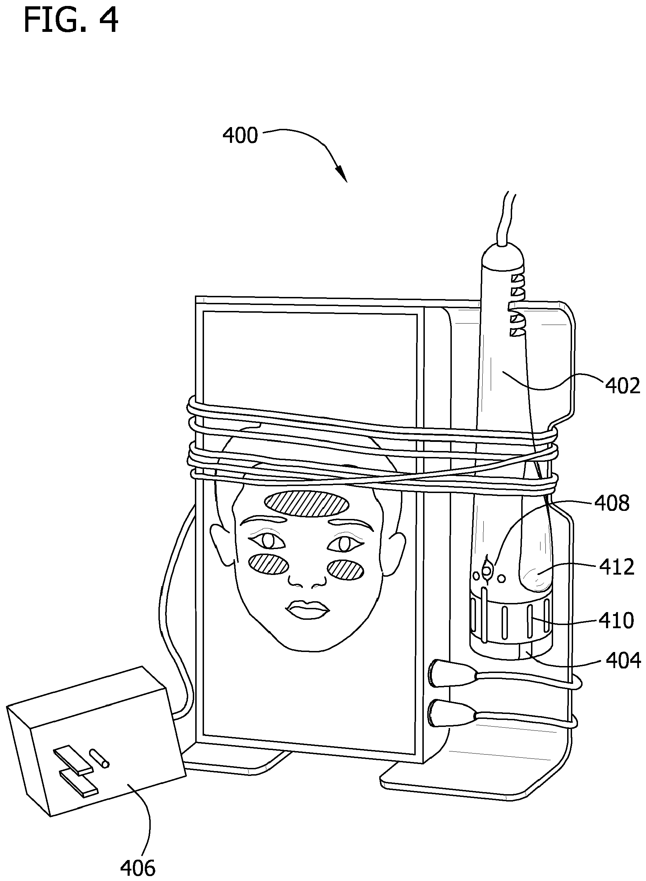

FIG. 4 is a perspective view of an alternative HFUS device 400 having the components of device 100 shown in FIG. 1. In the exemplary embodiment, the features of device 100 are integrated into a single handheld unit that is configured to treat infection within the subject. For example, device 400 includes a device housing 402, a treatment applicator 404, and a power source 406. Device housing 402 includes a user interface 408 similar to the first embodiment. In one embodiment, device 400 is configured to be hand-held and portable such that a user can utilize device 400 with one hand. In some embodiments, applicator 104 and/or housing 102 are configured to have an ergonomic design when held by a user, such as a design including one or more recesses 412 that aids in stabilization.

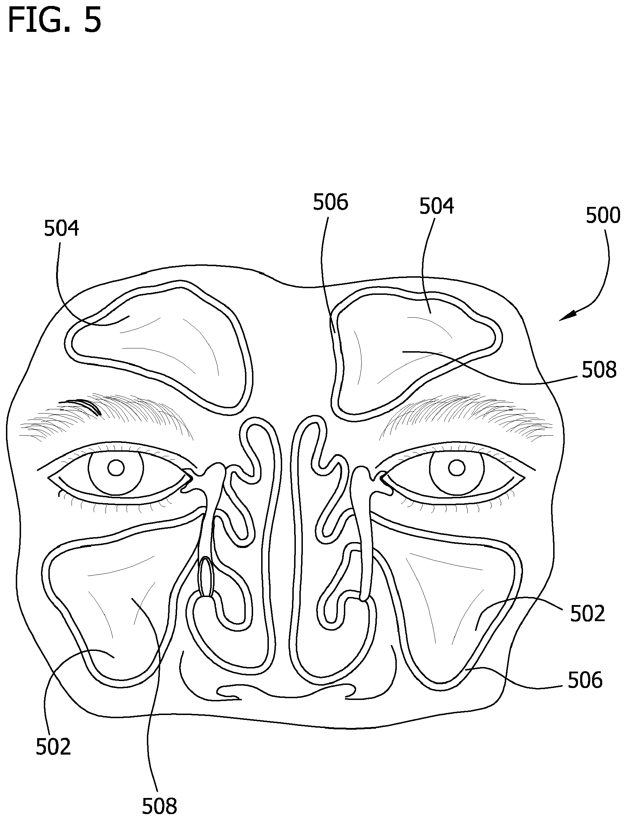

It should be noted that devices 100 and/or 400 are shown to be configured for treating infection in the sinus of a subject. Devices 100 and/or 400 are shown as being configured to treat treatment sites including the frontal 502 and maxillary sinus 508 (shown in FIG. 5) with the transducer delivering ultrasound energy through the skin and into the sinus cavity to treat infection.

**High frequency ultrasound (HFUS)**

To validate the effectiveness of treating infection with HFUS energy, testing of the output of device 100 shown in FIG. 1 was performed in a medical biofilms laboratory. As shown in more detail below, the output of device 100 was tested against (1) a methicillin-sensitive (MSSA) *Staphylococcus aureus* strain isolated from a sinus of a subject with chronic rhinosinusitis and (2) a methicillin-resistant (MRSA) *Staphylococcus aureus* strain isolated from a chronic wound of a subject. A CDC biofilm reactor (CDC-BR) was used to grow MSSA *Staphylococcus aureus* and MRSA *Staphylococcus aureus* biofilms on polycarbonate coupons that were subjected to testing.

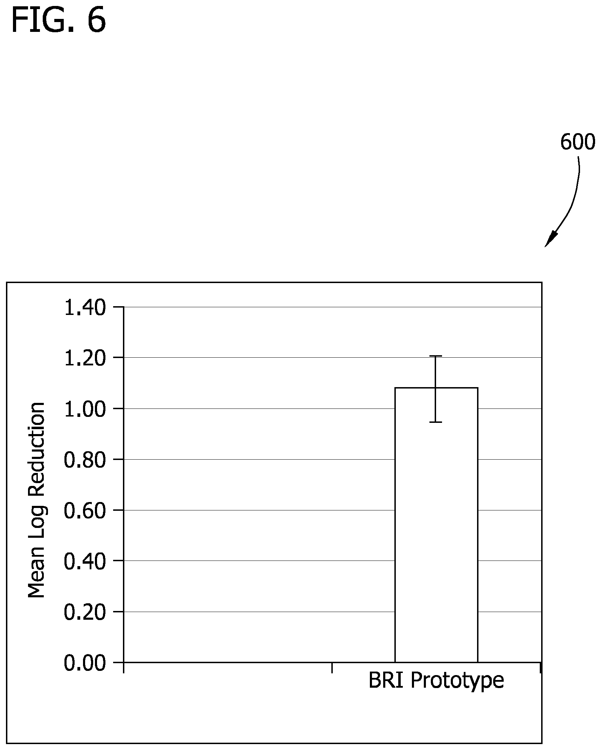

Test 1 - HFUS energy from device 100 was tested on MSSA *Staphylococcus aureus* coupons. The mean log density (MLD, $\pm$ standard deviation) of the control biofilms was $7.95\pm0.05$ log CFU/cm$^2$. Using device 100, coupons were exposed to five minutes of HFUS energy from applicator 104 at a frequency of 1 MHz and an intensity of approximately 1 W/cm$^2$. As shown by graph 600 in FIG. 6, treatment of the coupons with device 100 resulted in a mean log reduction (MLR) of $(1.08\pm0.13)$ yielding a 91.41% reduction of MSSA *Staphylococcus aureus* biofilm on the coupons.

To calculate the elimination of bacteria and/or biofilm in Test 1, the treatments were assessed relative to untreated controls using viable plate count methods. The coupons were placed in tubes containing 10 ml phosphate-buffered saline (PBS). A sequence of vortex, sonicate, and vortex was then used to remove bacteria from the coupons and produce a bacterial suspension. The suspension was serially-diluted in PBS and plated on Tryptic Soy Agar (TSA). The plates were incubated at 37° C for 24-48 hours and the number of colony forming units (CFU) was counted. Based on the dilution and the dimensions of the coupons, the CFU per unit area (CFU/cm$^2$) was calculated. The CFU/cm$^2$ counts were logarithmically transformed (base 10) to determine log density (LD) and a mean log density (MLD) was calculated from replicate coupons.

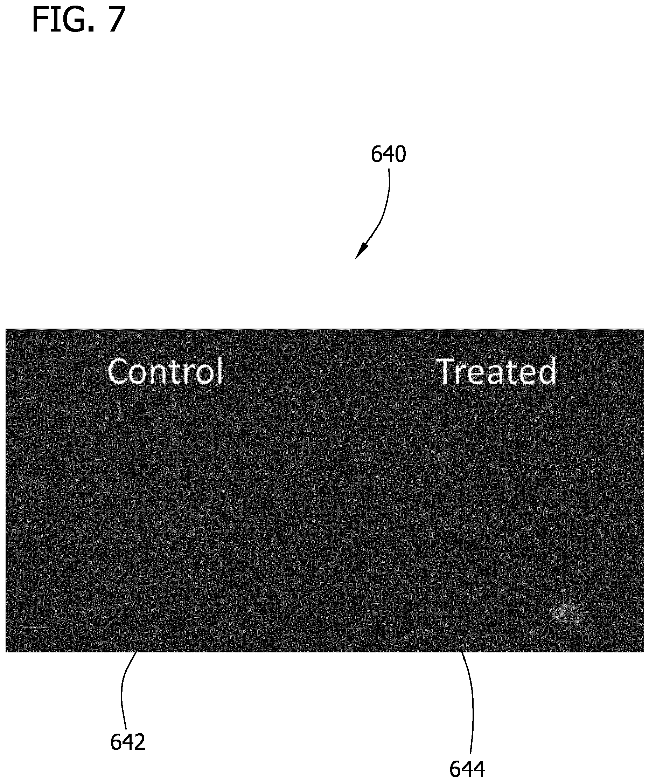

Test 2 - FIG. 7 displays microscope images 640 of the results of Test 2 using device 100 shown in FIG. 1. For Test 2, MSSA *Staphylococcus aureus* biofilms were grown in the CDC-BR, as described above, and the coupons were subjected to HFUS output from device 100 with a power level of 1 W/cm$^2$ (100%) at 1 MHz for 5 minutes. Two coupons were treated and one coupon served as an untreated control. After treatment, the coupons were treated with the LIVE/DEAD® BacLight™ Viability Kit which includes two nucleic acid stains, SYTO-9 and propidium iodide. SYTO-9 stains live bacterial cells green and propidium iodide stains bacterial cells with damaged membranes (dead cells) red. After treatment of the viability kit, coupons were imaged with a Leica SP5 confocal scanning laser microscope. As is shown by pictures 640, the coupon subjected to HFUS output 644 from device 100 had less infectious agents (e.g. bacteria and/or biofilm) than the control coupon 642.

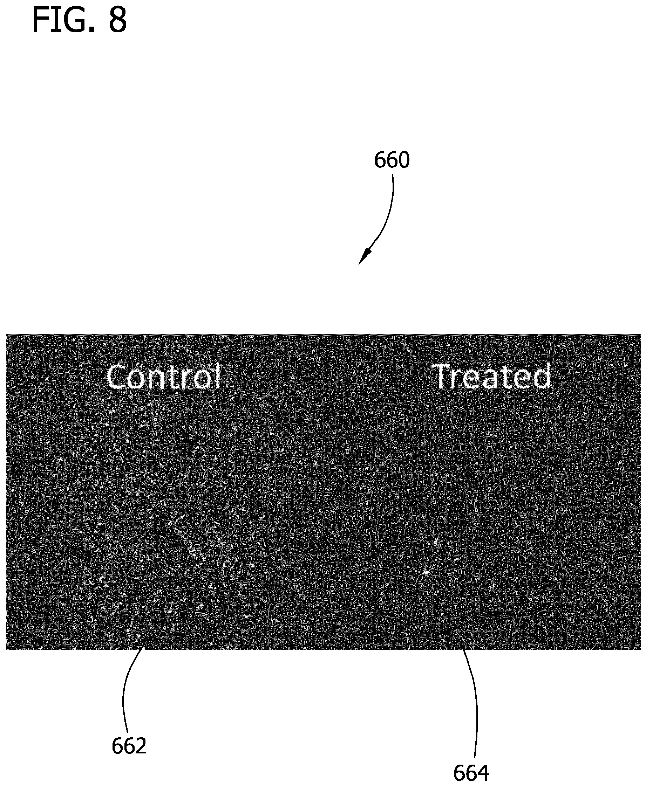

Test 3 - FIG. 8 displays microscope images 660 of the results of Test 3 using device 100 shown in FIG. 1. For Test 3, MRSA *Staphylococcus aureus* biofilms were grown in the CDC-BR, as described above, and the coupons were subjected to HFUS output from device 100 with a power level of 1 W/cm$^2$ (100%) at 1 MHz for 5 minutes. Two coupons were treated and one coupon served as an untreated control. After treatment, the coupons were treated with the LIVE/DEAD® BacLight™ Viability Kit which includes two nucleic acid stains, SYTO-9 and propidium iodide. SYTO-9 stains live bacterial cells green and propidium iodide stains bacterial cells with damaged membranes (dead cells) red. After treatment of the viability kit, coupons were imaged with a Leica SP5 confocal scanning laser microscope. As is shown by pictures 660, the coupon subjected to HFUS output 664 from device 100 had less infectious material (e.g. bacteria and/or biofilm) than the control coupon 662. As shown by the results of Tests 1-3, shown in FIGS. 6-8, device 100 is configured to negatively affect infectious agents inside the body. As described above and illustrated in FIGS. 6-8, the systems and methods described herein enable a user to treat biofilm. It is believed the output of ultrasonic vibratory energy at a frequency of 1 MHz has an effect on infectious agents and/or biofilms by turning infectious agents, such as bacteria, and/or biofilm into planktonic state, causing delamination of biofilm, creating a physical disruption, and/or breakdown of polysaccharides present in biofilm matrix.

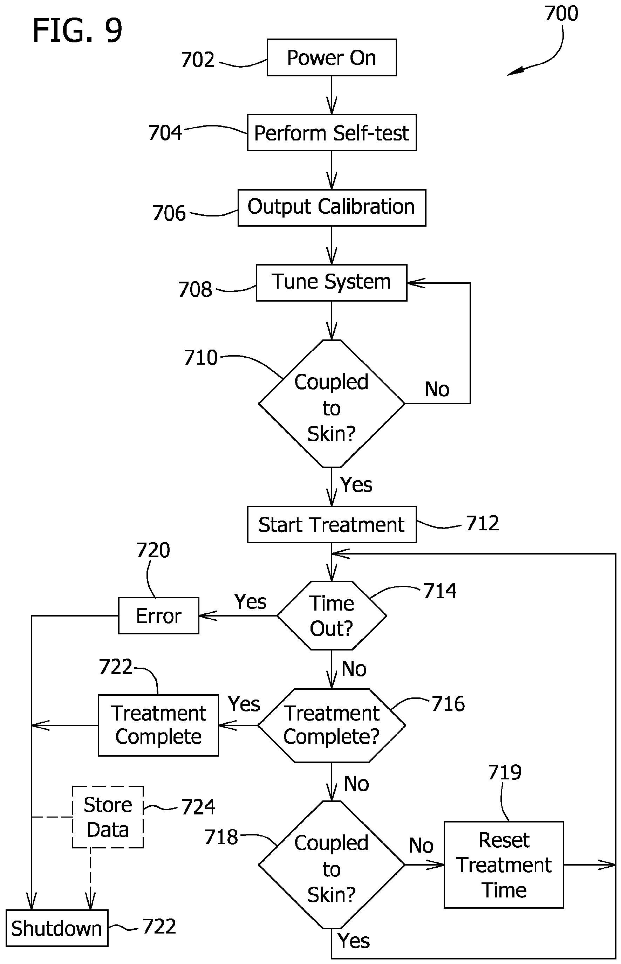

FIG. 9 is an exemplary flowchart of a method 700 for use with device 100 shown in FIG. 1. To initiate method 700, device 100 receives (e.g., at the power on step 702) instructions to start a treatment session from input interface 110 and/or presentation interface 108. Alternatively, treatment information can be transmitted in any known manner including through communication interface 108. In some embodiments, treatment information relates to a particular body area and/or region of the body to be treated. Alternatively, treatment information relates to the specific infection to be treated. After receiving instructions at step 702, device 100 performs a self-test at step 704. It should be noted that power and error checking algorithms performed during the self-test of step 704 can be implemented at any time throughout method 700. In the exemplary embodiment, processor 208 communicates with all hardware components to determine if any errors occur.

After performing a self-test at step 704, processor 208 calibrates the output of applicator 104 (step 706). In the exemplary embodiment, processor 208 tunes device 100 at step 708, based on the received treatment information. In one embodiment, tuning of device 100 is performed by determining a resonance (e.g. parallel or series) and locking into a frequency at the resonance selected. In one embodiment, tuning of device 100 at step 708 is performed when temperature sensor 320 detects a temperature that exceeds a predetermined threshold for applicator 104. Once device 100 is tuned (step 708), device 100 determines at decision block 710 if applicator 104 is coupled the skin of a user. To determine whether the applicator 104 is coupled to the user at 710, device 100 compares the impedance feedback received to a threshold that is correlated to applicator 104. In one embodiment, the impedance of applicator 104 when the applicator is coupled to skin is in the range of 100-500 ohms. Alternatively, the impedance range can be any range that correlates to the properties of the applicator 104.

After determining applicator 104 is coupled to the skin (decision block 710), device 100 starts a treatment session at step 712 and outputs energy (e.g. HFUS and tactile vibratory energy) through applicator 104 based on the received 702 treatment information. During the treatment session, processor 208 monitors device 100 to determine if device 100 has timed out (decision block 714), completed a treatment time (decision block 716), and/or determined that applicator 104 has been continuously coupled to the skin (decision block 718). It should be noted that processor 108 continuously determines if applicator 104 is coupled to the skin in the same manner as described in the determination step 710. If during a treatment session processor 108 determines that applicator 104 is not coupled to the skin, the treatment time is reset at step 719. The transducer may be held in position or signals could be applied to a treatment site for a few milliseconds, few seconds, or a few minutes before moving the transducer to a new location or altering the signals for application at a different treatment site.

This treatment protocol could be done robotically. In one embodiment, applicator includes a strap (not shown) that enables the device to robotically move within the strap. In an alternative embodiment, shell 306 is manufactured to be placed over a particular body part (e.g., knee, ankle, elbow, wrist, hand) and transducer 310 moves throughout shell 306 to provide ultrasound to varying locations. In such an embodiment, shell 306 would include castor oil, mineral oil, or any other non-conductive material that enables ultrasound transmission from transducer 310. As noted herein, device 100 can accomplish a virtual movement pattern by utilizing an array and selectively activating portions of the array to achieve the desired effect. In yet another embodiment, device 100 can be coupled to a robot configured to move over a portion of the body after being secured to the body. The device 100 could be used with timers by holding the applicator 104 at one position for a period of time and then moving it to another position. It could be simply isolated and rotated so that the ultrasonic frequencies or energy would be dispersed over a larger surface area through rotational movement. The output could be variable or continuous pulse. The output could be mobile, robotically positioned, or sequentially positioned for a time, distance, or specific angle location. This could be varied either remotely, via robot, or via control.

If processor 208 determines at decision block 714 a timeout has occurred, an error is provided to a user at step 720, device 100 is shutdown at step 722, and output through applicator 104 is stopped. Additionally, if processor 208 determines at decision block 716 that a treatment session is complete, the user is alerted that the treatment is complete at step 721 and device 100 is shutdown at step 722. In the exemplary embodiment, an alert is provided to a user visually (e.g. blinking light or changing light color) and/or audibly when an error occurs at step 720 or the treatment is complete at step 721. Alternatively, alerts at steps 720 and 722 can be provided to the user in any manner that facilitates notification to a user. In some embodiments, before device 100 is shutdown at step 722, all treatment session data is stored at step 724 in memory device 209 for transferring to another device.

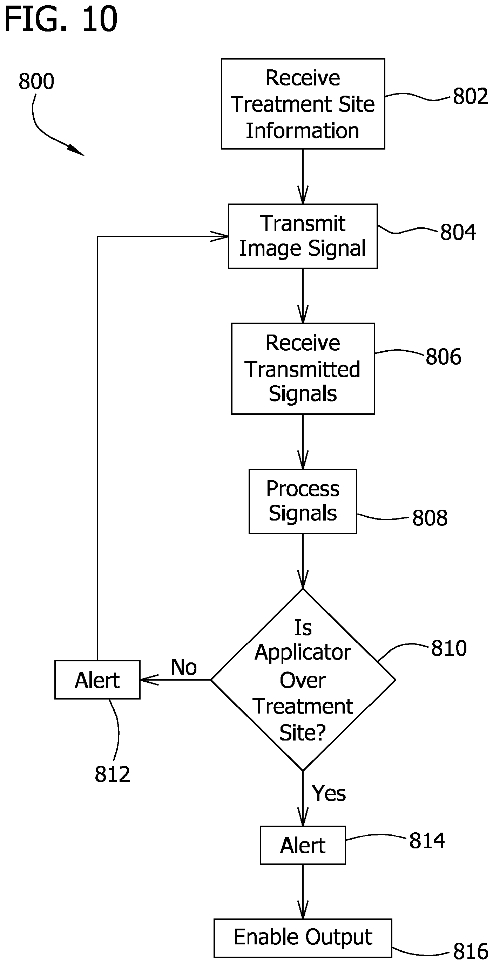

FIG. 10 is an exemplary flowchart of a method 800 for determining a location of device 100, shown in FIG. 1, in relation to a treatment site. To determine a location of device 100, a user selects a treatment site, such as by touching a graphical representation of the treatment site on display, and the selection is received at step 802. In the exemplary embodiment, a treatment site is selected through user interface 108 and transmitted to processor 208. The treatment site can be anywhere inside the body including, but not limited to, sinuses 502 and 504. Once the treatment site selection is received at step 802, imaging signals from imaging transducer 311 are transmitted at step 804 through applicator 104. In the exemplary embodiment, the imaging signals are pulsed ultrasound. Alternatively, the imaging signals can be any imaging signal that enables locating device 100 as described herein.

When imaging signals are transmitted in the body at step 804, they are reflected from objects (e.g. tissue) in the body, and are received by applicator 104 at step 806. In one embodiment, imaging transducer in applicator 104 transmits and receives the imaging signals described herein. Alternatively, the imaging signals are transmitted and received by a transducer in an array of transducers positioned in applicator 104.

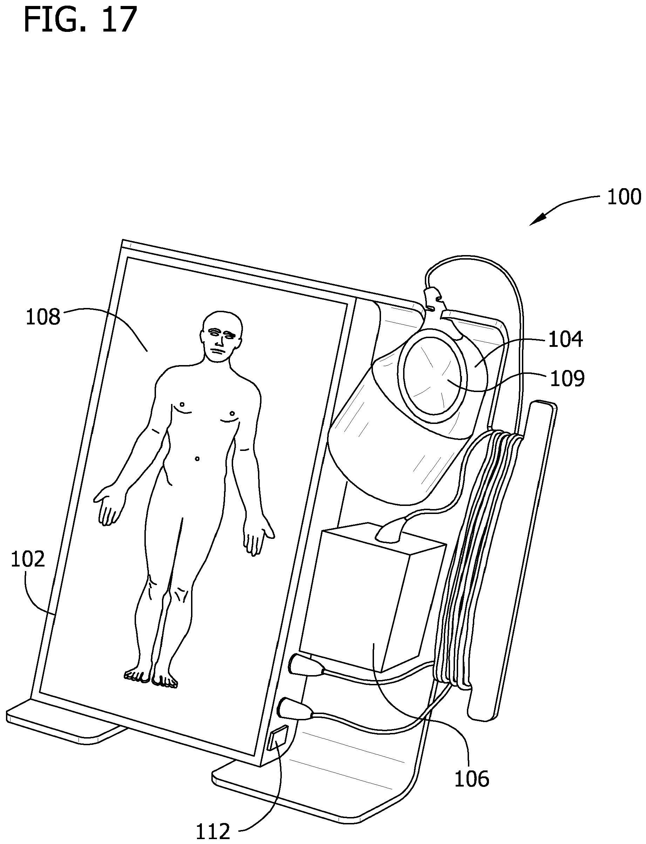

When the transmitted imaging signals have been received at step 806, the signals are processed by processor 208 at step 808. In the exemplary embodiment, the processed signals determine patterns of objects in the body and/or distances of the objects inside the body. The processed signals are then compared at decision block 810 to known patterns and/or distances of the received treatment site to determine if applicator 104 is over the treatment site. In some embodiments, processor 208 performs the comparison 810 by determining if the processed signals are within a predetermined threshold of known and/or stored information about the treatment site. As shown in FIG. 17, processor 208 may be configured to generate a graphical image of a body and indicate on the graphical image the location of applicator 104.

**EP 3 278 839 A1**

If processor determines at decision block 810 that applicator 104 is not over the selected treatment site, the user is alerted at step 812 and image signals are transmitted at step 804 again. If processor determines at decision block 810 that applicator 104 is over the selected treatment site, the user is alerted at step 814 and treatment modalities of device 100 are enabled to be output at step 816. In the exemplary embodiment, alerts at steps 812 and 814 are provided to the user through user interface 108. In the exemplary embodiment, alerts at steps 812 and 814 are visual. Alternatively, alerts at steps 812 and 814 can be communicated to a user in any manner that facilitates notification as described herein including, but not limited to, auditory signals and/or tactile feedback sent from device 100. In one embodiment, the step of sending the alert 812 includes providing the user the determined location of applicator 104. For example, if a user selects the knee as a treatment site and the applicator is positioned over the tibia, the user would visually see that the applicator is not over the selected treatment site and that it is on the lower leg. It should be noted that method 800 could be utilized anywhere throughout method 700 shown in FIG. 10. In another embodiment, a marker (i.e., a detectable device) may be provided on an implant or within a desired treatment site. The marker may be detectable by device 100. For example, the marker may be an RFID tag or magnetic tag or other component detectable by a sensor. Device 100 may include a detector for detecting the marker to determine if applicator 104 is correctly positioned for treating the treatment site. In one example, the marker may be biodegradable and/or degradable based on use and treatment. For example, the marker may be degradable by ultrasound such that after the marker is subjected to a certain amount of ultrasonic treatment using device 100, the marker is no longer detectable by the device. In this example, the degradation of the marker signifies that treatment has been completed.

The methods and systems described herein can be utilized to treat infections anywhere inside the body. In one embodiment, the methods and systems described herein are utilized to treat sinusitis. In one example, device 100 provides treatment of infection in the sinus using applicator 104 with method 800 by treating the frontal sinus 502 with ultrasound energy having a frequency of 1 MHz and an intensity of 0.5 W/cm$^2$ for 2 minutes, and the maxillary sinus 504 with ultrasound energy having a frequency of 1 MHz and an intensity of 1.0 W/cm$^2$ for 2 minutes. The treatment time, frequency and power of the applied ultrasound energy can vary depending on specific types of infections.

In one embodiment, method 700 and/or 800 can be utilized to determine if there has been a buildup of fluid in and/or around a portion the body, such as sinus 500 shown in FIG. 5. For example, during acute and chronic sinusitis there is a buildup of fluid in sinus cavity 502 and/or 504. When a sinus cavity is void of fluid, the imaging ultrasound signal will reflect off of an anterior side 506 of the cavity and not propagate due to the air in the cavity. When there is infection (e.g. sinusitis), which often leads to the presence of fluid (e.g. mucus), the fluid will allow the imaging signal to propagate to a posterior wall 508 of the sinus cavity and reflect an echo. In such an embodiment, device 100 can be optimized to compensate for the fluid in the sinus cavity 500 and provide output energy that will be transmitted to both sides 506 and 508 of the sinus cavity 500. To accomplish this, as described above, separate transducers with varying signals or components of a transducer array could be utilized.

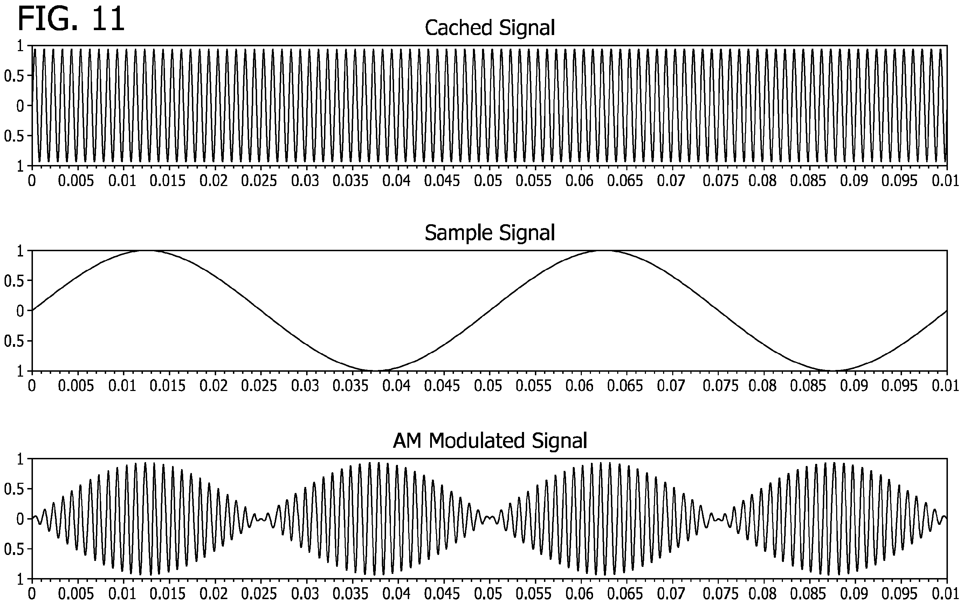

In some embodiments, the HFUS energy from applicator 104 is modulated. In such an embodiment, a narrow beam of ultrasound (carrier) is amplitude modulated (AM) with an audio signal which creates a narrow beam that can only be heard along the path of the beam, or from objects in the path of the beam. Air has non-linear acoustic properties that cause the signal to self-demodulate over the path of the beam (shown in FIG. 11).

The concept of self-demodulating AM signals from non-linearities in the transmission media can be applied to the problem of getting optimal LFUS frequencies for biofilm and bacteria reduction to the sinus cavities via HFUS waveforms. As ultrasound travels through different materials, the wavelength ($\lambda$) is determined by the speed of sound ($c$) through the media the waveform is traveling through divided by the frequency of the waveform ($f$), as shown by the following equation:

$$\lambda = {^c}\!/\!_f$$

The speed of sound (c) for various materials is shown in the table below.

| Material | Velocity (m/s) |
|---|---|
| air | 331 |
| fat | 1450 |
| water (50 C) | 1540 |
| human soft tissue | 1540 |
| brain | 1541 |
| liver | 1549 |

(continued)

| Material | Velocity (m/s) |
|---|---|
| kidney | 1561 |
| blood | 1570 |
| muscle | 1585 |
| lens of eye | 1620 |
| skull-bone | 4080 |
| brass | 4490 |
| aluminum | 6400 |

By using a waveform that is optimized for bacteria and biofilm removal and taking advantage of the non-linearities caused by the change in the speed of sound at the interface of different biologic materials, device 100, and more specifically transducer 310, is configured to treat infections with optimal waveforms, as well as manage the pain and discomfort associated with the condition while ensuring patient safety during the treatment, using the information below. To ensure patient safety and comfort, the carrier signal is selected to be above 20 kHz, with the optimal frequencies being between 1MHz - 3 MHz.

In one embodiment, the algorithm utilized for treatment modulates the treatment signal over a 1-second (1000 mS) period. If pulsed ultrasound treatment (PUS) is used to minimize heating, a typical pulse ratio is 1:9, meaning the ultrasound output would be active for 1 ms and off for 9 ms. Each pulsed cycle would take 10 ms.

$$\frac{1000\ mS}{10\ mS} = 100\ frequency\ steps$$

Assuming the desired frequency range for the treatment signal is 20 kHz - 80 kHz, the frequency step would be calculated as follows:

$$\frac{80\ kHz - 20\ kHz}{100\ steps} = 600\ \text{Hz/Step}$$

In this example, the carrier frequency would be Hz1MHz, and the treatment signal would start at 20 kHz. After every pulse cycle of 10 ms, the frequency of the treatment signal would be increased by 600 Hz. Once the upper limit of the treatment signal frequency is reached, in this example 80 kHz, the algorithm would be repeated starting at the start frequency until the desired total treatment time is reached. In one embodiment, the acoustic output will have a resonant frequency at 1 MHz and the AM envelope at a frequency = $20\ kHz + n * 600\ Hz$. Where n = loop count, and n < 100.

With this algorithm, each selected treatment envelope frequency is output for the same length time, but the actual number of periodic waveforms would vary with the frequency being used. At the lowest treatment frequency, 20 kHz, each envelope period is 0.05 ms. This gives 20 periods of this modulated treatment signal over this (0.1 ms) frequency step. At the highest selected treatment frequency, 80 kHz, each period is 0.0125 ms, giving 80 period of the modulated treatment signal over this (0.1 ms) frequency step. The algorithm could be adjusted so that each desired treatment frequency is active for the same number of periods of the modulated signal, instead of the same amount of time. It is contemplated that the output could be continuous ultrasound (CUS) instead of PUS.

In one embodiment, the algorithm is modified so that the amount of ultrasound envelope determines the length of the treatment envelope, rather than a predetermined time. In cases where the user is applying a coupling gel to the surface of the skin prior to treatment, it is contemplated that once applicator 104 is in contact with the treatment site, the amplitude of the carrier frequency could be gradually increased prior to treatment to allow bubbles in the coupling gel to degas or dissipate. It is also considered that suction, or negative pressure, could be used to achieve better coupling of applicator 104 with the skin.

In one embodiment, a simple graphic on the presentation interface 104 indicates the area to be treated. For example, a picture of a face could be shown with sinus areas capable of illumination. If the setting for the frontal sinuses is selected, LEDs behind the frontal sinus area would illuminate. If the maxillary sinus is selected, LEDs behind the maxillary sinus area would illuminate. The optimal time, power, and modulation schemes would be selected based on the area to be treated. The display could also be implemented with an LED, VFD, or other methods known in the art.

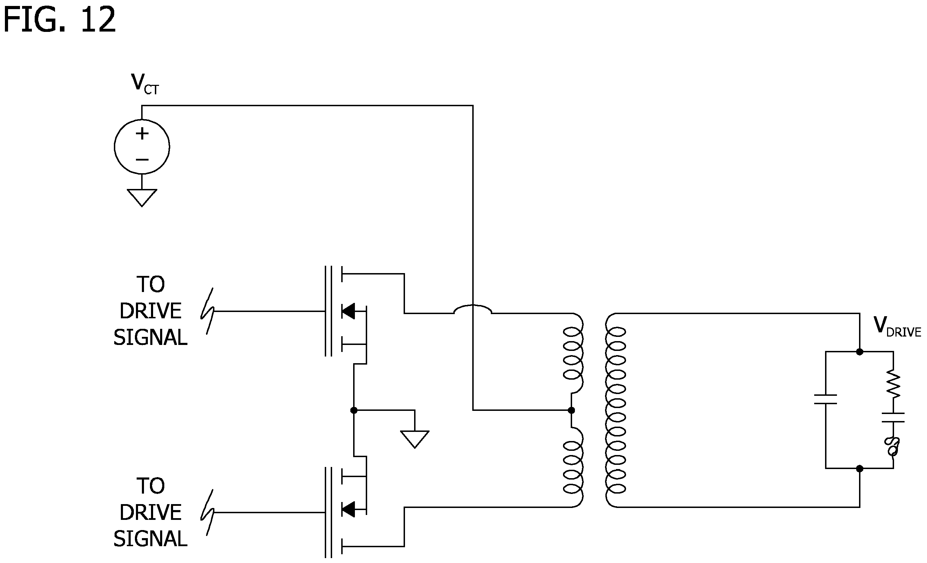

Referring to Fig. 12, a traditional center tapped transformer in a push pull configuration can be used to implement aspects of a modulation scheme. The voltage to the center tap could be configured to oscillate at the target treatment frequency while the carrier frequency would be pulsed to the push-pull FETs. Other analog and digital methods of creating the modulated signal which are known in the art could be implemented as well.

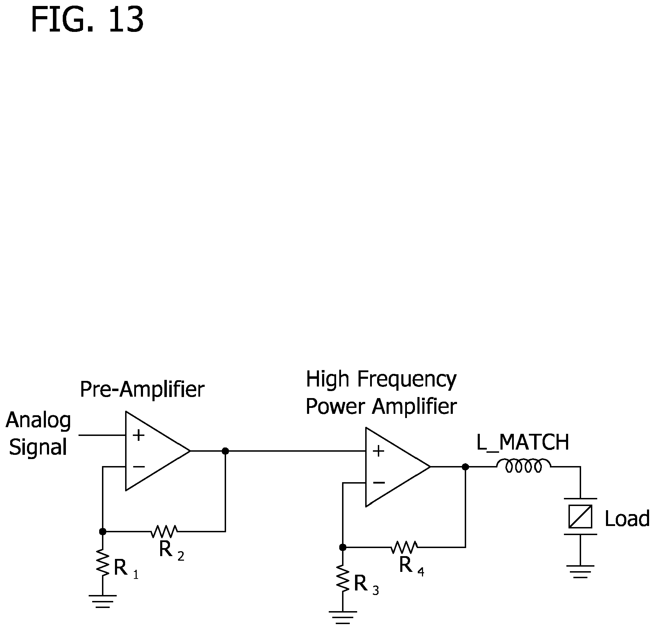

Referring to FIG. 13, in another embodiment, a waveform generator and high frequency power amplifier configuration implements the modulation. The modulated signal, possibly supplied by a processor, would be fed into the pre-amplifier with a predetermined gain of Av = 1+ (R2/R1). The amplified signal would then feed into the high frequency power amplifier, which would amplify the signal again with the predetermined gain of Av = 1 + (R4/R3). When driving a piezoelectric transducer, a matching inductor is added in series to the load to compensate for the capacitance of the load.

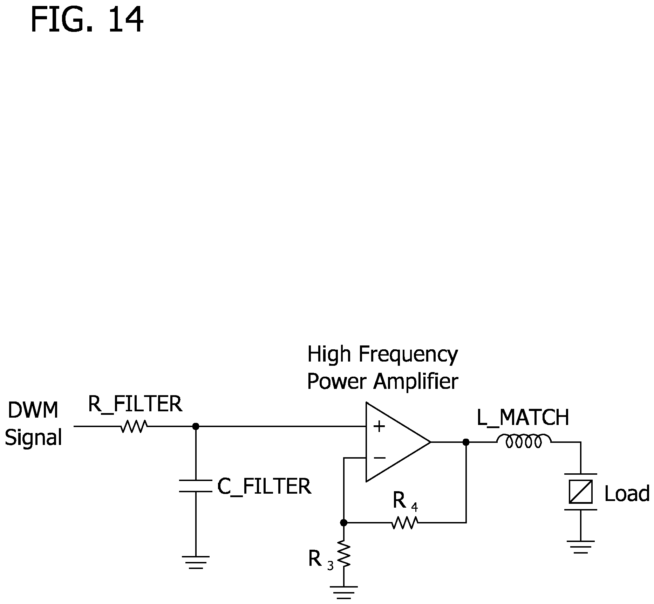

Referring to FIG. 14, in another embodiment, a pulse width modulated signal and high frequency power amplifier configuration implements aspects of the modulation (shown in FIG. 14). A pulse width modulated signal, possibly supplied by a processor, is fed into an RC filter. The RC filter acts as a digital to analog convertor and convert the pulse width modulated signal into an analog signal. The cut-off frequency of the RC filter is determined by the equation Fc = 1/(2*Pi*R*C). The converted analog signal is then fed into the high frequency power amplifier with a predetermined gain of Av = 1 + (R4/R3). When driving a piezoelectric transducer, a matching inductor is added in series to the load to compensate for the capacitance of the load.

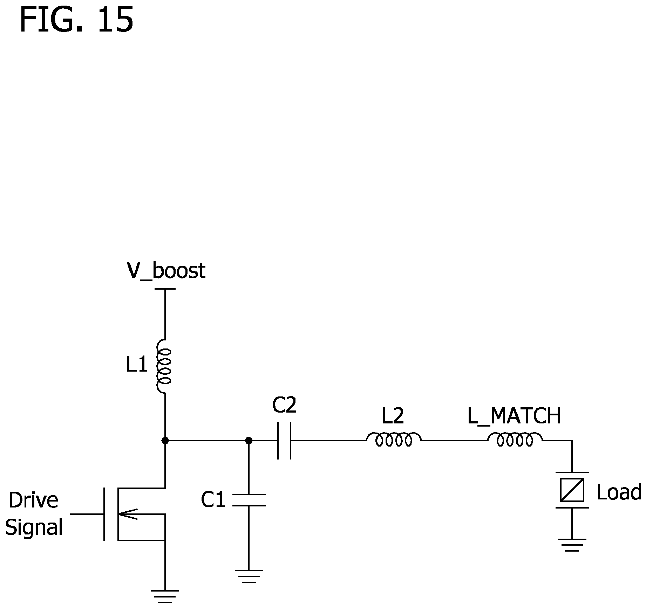

In another embodiment illustrated in Fig. 15, a Class E amplifier is used to implement aspects of the modulation. The Class E amplifier is suitably designed specifically for the drive frequency and N-Channel Mosfet that is defined for the system. The drive frequency is determined by the desired frequency to drive the load. The values of R_Load, L1, C1, C2 and L2 are determined using the following equations:

$$R_{Load} = \frac{(Vcc - Vo)^2}{P} * 0.576801 - (1.0000086 - \frac{0.414395}{QL} - \frac{0.577501}{QL^2} + \frac{0.205967}{QL^3})$$

$$C1 = \frac{1}{34.2219 * f * R} * \left(0.99866 + \frac{0.91424}{QL} - \frac{1.03175}{QL^2}\right) + \frac{0.6}{(2 * Pi * f)^2 * L1}$$

$$C2 = \frac{1}{2 * Pi * f * R} * (\frac{1}{QL - 0.104823})(1.00121 + \frac{1.01468}{QL - 1.7879} - \frac{0.2}{(2 * Pi * f)^2 * L1})$$

$$L2 = \frac{QL * R}{2 * Pi * f}$$

When driving a piezoelectric transducer, a matching inductor is added in series to the load to compensate for the capacitance of the load.

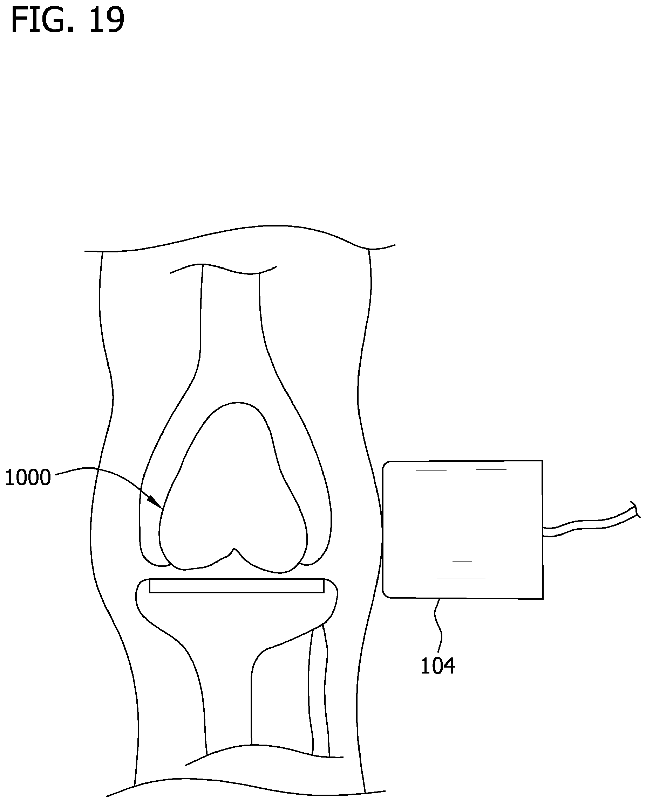

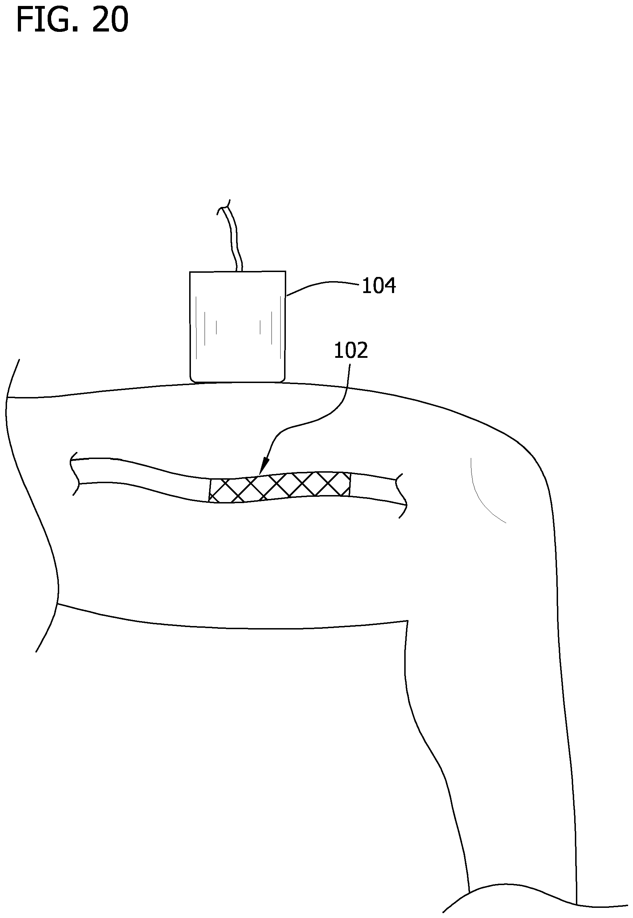

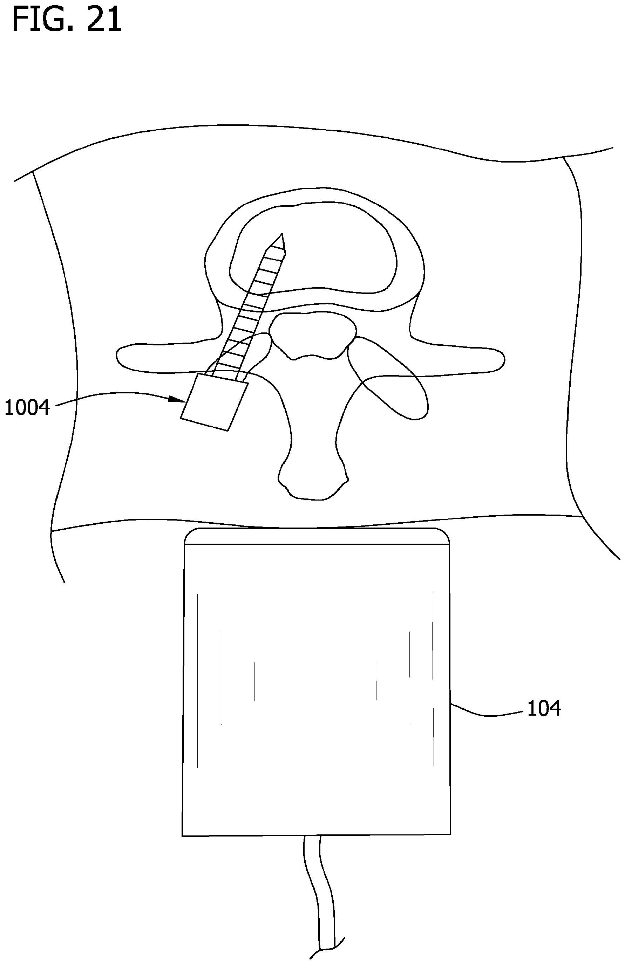

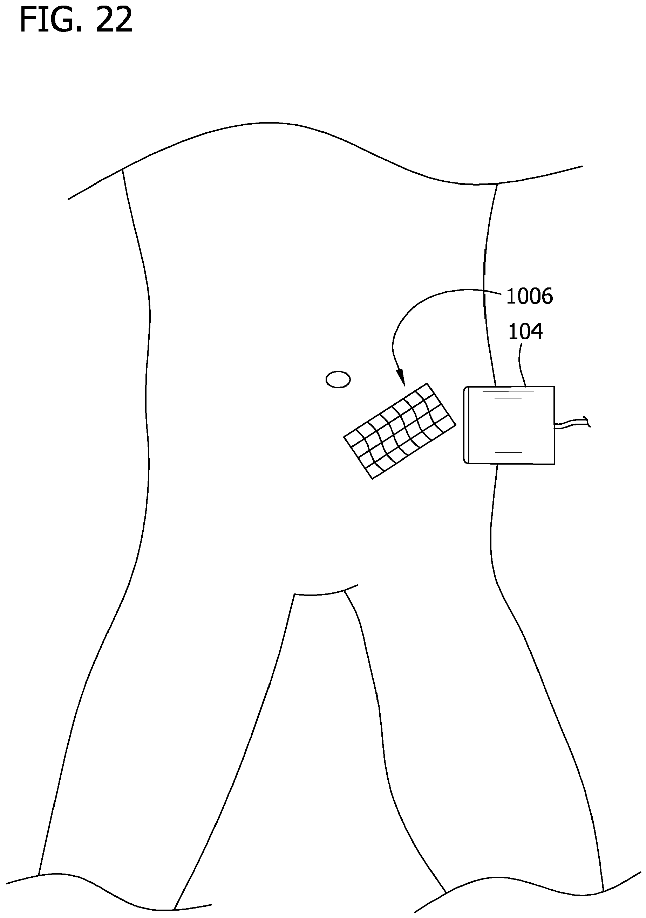

While some methods and systems have been described in treating infection in the sinus, it is also contemplated that the methods and systems described herein could be used to treat infection in other sub-dermal and dermal treatment sites including, but not being limited to, post-operative sites, joint replacements, metallic implants, polymeric implants, cystic fibrosis, skin ulcers, on and/or around the ear (e.g. otitis), infected nails, endothelium, vascular, or any passageway (i.e. bronchi, joint space synovium or intraarticular space, peritoneum, pleura, or prostate). For example, as shown in FIG. 19, device 100 can be used to treat infection for a knee replacement 1000 implanted in a subject's body. The components of knee replacement 1000 for treatment by device 100 may include metal components, polymeric components, biologic components, and mesh components, among other types of components. In another example, shown in FIG. 20, device 100 can be used to treat infection at a stent 1002 (e.g., peripheral stent, as shown, or coronary stent, or neurovascular stent) implanted in a subject's body. The components of stent 1002 for treatment by device 100 may include metal components, polymeric components, biologic components, and mesh components, among other types of components. In yet another example, shown in FIG. 21, device 100 can be used to treat infection at a screw 1004 (e.g., spinal screw, as illustrated, or other fastener) implanted in a subject's body. The components of screw 1004 for treatment by device 100 may include metal components, polymeric components, biologic components, and mesh components, among other types of components. In yet another example, shown in FIG. 22, device 100 can be used to treat infection at a mesh 1006 implanted in a subject's body. The components of mesh 1006 for treatment by device 100 may include metal components, polymeric components, biologic components, and mesh components, among other types of components.

In one embodiment, device 100 is utilized to prevent capsular contracture. Capsular contractures occur when the collagen-fiber capsule formed around a foreign material implanted in the human body tightens and is squeezed together. The foreign material can include, but is not limited to, breast implants, artificial pacemakers, and orthopedic prostheses. It has been shown that bacterial biofilms on foreign material implanted in the body may cause chronic inflammation and contribute to this condition. Device 100 can be utilized to prevent the capsular contracture by treating biofilm, treating scar tissue, and increasing blood flow.

Included within the treatment of biofilms are methods to prevent the formation biofilms. This could be done by using device 100 in the hand held form, or by integrating the device into existing medical devices such as surgical dressings, wraps, continuous passive motion devices, wound vacs, and adhesive bandages. In such an embodiment, applicator can be fabricated to have at least a portion that is flexible.

In one embodiment, local pressure is combined with the output of device 100 to enhance the effect. In such an embodiment, device 100 is configured to be inserted into and/or a portion of device 100 (e.g. applicator 104 or transducers) is configured to be integrated into a device that applies manual pressure or a compressive force (e.g., a blood pressure cuff). In the exemplary embodiment, a device that applies manual pressure is an orthosis for joint rehabilitation, including but not limited to, an orthosis from Joint Active Systems of Effingham, Illinois. Devices may include a pressure sensor which would guarantee that a correct pressure is applied to external or internal applicators or transducers. The manual or compressive force would stabilize the device 100 against dynamic or pulsatile movement of the body tissue, which could affect location or position of the applicator or transducer.

Non-linearities and impedance mismatches can also be introduced into the body by injecting contrast agents, micro bubbles, fluids, and/or air. Device 100 can be utilized to apply ultrasound and micro bubbles to prevent the formation of scar tissues. In one embodiment, device 100 is configured to enhance and/or eliminate the use of micro-bubbles. By taking advantage of an acoustic mismatch or non-linearity at the interface of the bubbles, the signal could be modulated to enhance the therapeutic action of the micro bubbles. If there was enough of a mismatch at the interface at the area of targeted scar tissue, the use of micro-bubbles might not be required.

The acoustic mismatch described above can enhance the delivery of pharmaceuticals. In one embodiment, the methods and systems described herein are optimized for the selective rupture of cell membranes to enhance pharmaceutical efficacy. Pharmaceuticals could also be designed to have a specific resonance which could be excited to enhance delivery or cell rupture. The pharmaceuticals might be engineered to provide a large acoustic impedance mismatch. The signal could also be optimized to enhance the movement of pharmaceuticals across the blood brain barrier.

To aid in the disbursement and/or absorption of pharmaceuticals to increase a pharmaceutical's efficacy, the ultrasound output of device 100 can heat tissue as a result of vibratory energy. This heating can allow cellular absorption of pharmaceuticals to increase. Additionally, removal and/or elimination of a portion or all of bacteria and/or biofilm in a location will affect the pH of the tissue in and around the treatment site. As such, the changing pH of the tissue can enable increased absorption of pharmaceuticals. This could be used when delivering chemotherapy, vasodilators, bronchodilators, and hyaluronic acid. Accordingly, the device 100 may include one or more modalities for specific use in combination with pharmaceutical treatment to enhance the pharmaceutical treatment. The modality is configured to output desired ultrasound to increase a pharmaceutical's efficacy. As an example, the cell membrane and cell wall are rigid structures which prevent certain pharmaceuticals from passing into the cell (e.g., virus, parasites, and/or bacteria). For example, methicillin-resistant staph may be bacteria that function to resist drug effects by altering the cell wall/membranes of a non-resistant staph to prevent a drug from getting through the membrane. The present device 100 may include one or more modalities for delivering ultrasound configured to make the cell wall/membrane more porous so that drugs can easily enter into the cell to enhance treatment. The ultrasound may be applied in conjunction with adjusting the local pH to make a more alkaline environment. For example, a more alkaline environment may make the pharmaceutical agent more effective, especially in combination with ultrasound treatment.

In yet other embodiments, the device may include modalities for enhancing the effectiveness of other treatments, including but not limited to chemotherapy, stem cell therapy, biologic cell therapy, enzyme therapy, grafts (e.g., allograft and autograft), and biopharmaceuticals. Each modality may be specific to a certain additional treatment to produce a synergistic effect. That is, each modality is configured to output desired ultrasound that enhances the effectiveness of the particular treatment. For example, device 100 may be configured to deliver ultrasound to enhance tissue ingrowth or biological response to a graft (e.g., cartilage graft or cell graft). In a particular example, ultrasound from device 100 may be delivered to a scaffold made in accordance with U.S. Patent No. 7,299,805, the entirety of which is incorporated by reference herein, to enhance the scaffold ingrowth fixation or stability. Ultrasound from device 100 could be used during the time of implantation to inhibit infection or enhance ingrowth due to its improved vascular function, and it could also enhance the pharmaceutical effects locally.

Device 100 may include one or more modalities for improving fluid flow of medicinal agents (e.g., pharmaceuticals, biologics, enzymes, etc.) into cells. For example, a modality of device 100 may be configured to make viscous fluid less viscous, thereby affecting cell walls or cell membranes, for example, by making previously resistant cell walls porous to medicinal agents. The agents may become more sensitive due to the fact the cell walls or cell membranes may be porous.

The systems and methods described herein can also be utilized with modalities to clean and/or eradicate residual debris resulting from the effects of the output of device 100. For example, a pulsed lavage can be used after treatment with device 100 to clean debris. In the example of sinus treatment, a user could utilize a nasal spray before, during, or after treatment of device 100 on a sinus treatment site. The spray would allow mucus positioned in the nasal cavities to escape quickly.

It is also thought that the parasitic diseases such as trichinosis, scabies, and toxoplasmosis could be treated with the methods and systems described herein. These parasites create spores, which are very resilient and difficult to treat. It is thought that these types of parasitic diseases may be treated by targeting the resonance of the spores and/or the acoustical impedance mismatch from the spore to the surrounding materials. Thus, the waveform can be optimized to break up the spores so they can be treated with pharmaceutical agents.

The methods and systems described herein may be used to optimize flow of fluid media by selecting a modulation frequency that is optimized to the media targeted for manipulation. This could be used for in-vivo applications such as blood flow in vessels or arteries. In one example, methods and systems could also be used for myocardial infarction to improve blood flow through an artery, for example, and/or to produce more laminar blood flow. If there is disruption or spasticity, ultrasound from the device 100 could be delivered to the location of the disruption that causes a heart attack or the location of a vasospasm. In another example, with respect to blood flow, the device 100 may include one or more modalities for increasing $O_2$, white blood cells, and/or nutrients to tissue. Ultrasound delivered by device 100 may enhance the body's normal response. A modality of device 100 may also make greater vascular permeability to allow greater oxygen tension, greater white blood cell delivery, and blood flow to specific areas. It can dilate the blood vessel as well. For example, a modality of device 100 causes delivery of ultrasound that increases blood flow or vascular dilatation or vascular permeability to deliver more nutrients, white blood cells, and oxygen, which enhances the normal body response and its efficacy. Depending on the configuration of the waveform, it could be used to cause or prevent constriction or spasms in the vessels. In one embodiment, device 100 is utilized to heat mucus in the body enabling the mucus to flow.

An ultrasound waveform could also be used in medical devices and instrumentation to enhance the flow of fluids and pharmaceuticals. Also industrial applications such as injection molding, oil pipelines, gasoline and biofuel production and transport could benefit. This could be done with injection molding for fracking of oil/gas with or without agents to enhance the viscosity of gas, oil, etc. Ultrasound could be applied to move, suction, or break up debris at the fracking tip so that fluid would flow rather than be obstructed by material, sand, etc. The methods and systems described herein could be used in conjunction with the drag modulation techniques described in U.S. Patent No. 6,842,108 to Peter M. Bonutti, the contents of which are incorporated by reference herein in their entirety. Combining elements of the techniques enables a more efficient reduction of drag, fluid flow, and acoustic disturbance. The device 100 could be used to eliminate bubbles, separate particles from liquid, and/or further pack or compress materials as needed. This could be used to separate materials of different viscosities (e.g., oil, water, polymer metal or different types of metals). The device could be used for molding and manufacturing as well as inside the human body.

The systems and methods described herein can be used with mechanical agents, such as pseudoephedrine or other vasodilators or water. Moreover, the systems and methods may be used to enhance delivery of Cannabidiol (CBD) and Tetrahydrocannabinol (THC) for pain via sonophoresis. The device 100 may increase the transdermal absorption of semisolid topical compounds. This method of delivery will allow localized delivery of the medication, potentially eliminating the need for systemic delivery. This method could potentially maximize the effectiveness of pain treatment while minimizing the psychoactive effects. The method may include the use of vasodilators, solvents, thermal optimization, and/or pH optimization to enhance efficacy.

The acoustic mismatch between biologics in the body may allow for the separation of (e.g., delamination of) biologic and/or non-biologic materials throughout the body based on the modulation scheme that is selected. This could allow for acoustical separation of dissimilar materials including but not limited to mucus, calcium deposits from vascular system or articular cartilage, earwax, or the treatment of chondrocalcinosis. As such, varying frequencies can be transmitted into a treatment site to affect attachment to different surfaces. For example, for use after a knee arthroplasty, device 100 could transmit a first frequency to affect bacteria or biofilm attachment to a native surface (i.e. bone or soft tissue) and a second frequency to affect bacteria or biofilm attachment to a foreign surface (e.g. metal or plastic). This could be done with pulsatile fluid suction and irrigation. This could also be done with subsonic, EMR, radiation, or electroshock therapy.

This technology could be a part of a multimodal treatment with any combination of ultrasound, vibratory, pulsed electromagnetic fields (PEMF), electroshock, pharmaceutical, phototherapy, or thermal treatments.

The systems and methods can also be used to dilate the cell membrane coatings. The vibratory frequency could optimize cell wall/membrane permeability to enhance local effect. This could also be used for autoimmune disease to enhance the effect. For example, if a joint has rheumatoid arthritis, one could use ultrasound and the thermal effect could decrease symptomatology and decrease fluid flow as well as enhance and localize the effect of the pharmaceutical agents from inflammatory diseases at specific locations, i.e. skin, joint, lungs, sclarea, derma, etc. This could also be done in combination with the treatments to enhance flow. For example, if one has pulmonary hypertension in later stages because of pulmonary fibrosis, ultrasound treatment could enhance airflow through the lungs as well as vascular permeability or vascular flow so one would decrease the pulmonary hypertension. Areas of pulmonary sclerosis could be used as an adjutant for pharmaceutical management to decrease the degree of pulmonary hypertension.

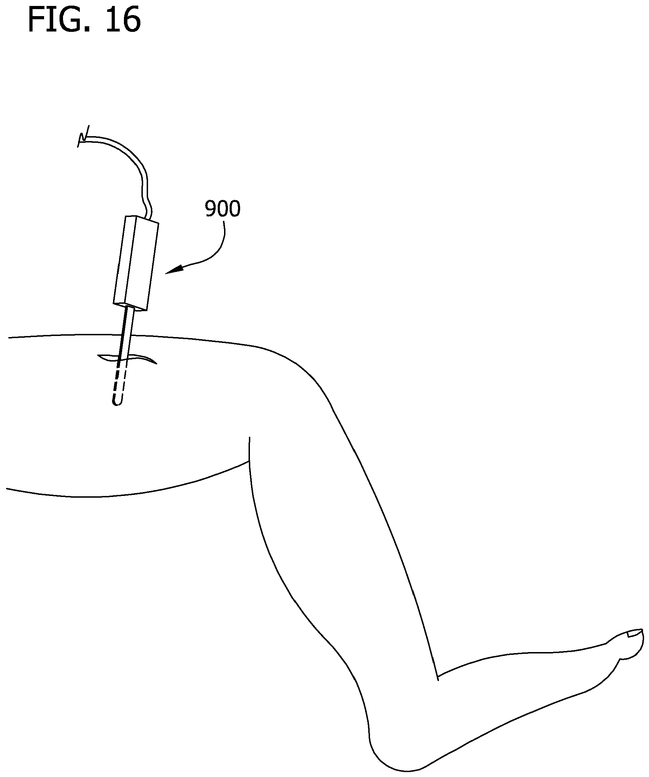

It should be noted that the systems and methods described herein are not limited to transdermal use. FIG. 16 is a perspective view of an alternative HFUS device 900 having the components of device 100 shown in FIG. 1. In the exemplary embodiment, the features of device 100 are integrated into a single handheld unit that is designed for an intracorporeal and/or percutaneous use to affect bacteria, biofilm, and/or infection within the body. In such an embodiment, at least a portion of applicator 104 is located and/or positioned in the body to provide HFUS. However, it should be noted that any portion of applicator can be inserted in any body portion to provide HFUS. The applicator 104 could be placed in body orifices, through an incision, or through cannula or expanding cannula.

In one embodiment, device 100 may include a light device, an endoscope, a fiber optic device, and/or other medical viewing devices to aid in viewing the treatment site inside the subject's body. In one example, the medical viewing device may be associated with applicator 104. In one particular example, applicator 104 may be provided on a distal end of a catheter, which includes the medical view device, for insertion into the subject's body. In other examples, the medical viewing device may be separate from applicator 104. Device 100 may include a specific modality for viewing of the treatment site using the viewing device. Ultrasound could also mark tissue to allow visualization of the cells or tissue during an endoscopic procedure. In addition, additional imaging may be used in conjunction with device 100, such as but not limited to MRI, CT, and PET scans of the treatment site.

In one embodiment, device 100 may include a fluid delivery device for delivering fluid to the treatment site. For example, the fluid delivery device may be configured for irrigating the treatment site and/or delivering pharmaceuticals or other substances to the treatment site. In one example, the medical viewing device may be associated with applicator 104 such that the applicator and the fluid delivery device can be used simultaneously or during the same treatment. In one particular example, applicator 104 may be provided on a distal end of a catheter, which includes the fluid delivery device, for insertion into the subject's body. For example, where the modality of device 100 causes delamination of bio film from an implant or other surface, the fluid delivery device can be used to remove the delaminated biofilm from the body. In other examples, the fluid delivery device may be separate from applicator 104. Device 100 may include a specific modality for delivery of fluid to the treatment site using the fluid delivery device.

In one embodiment, device 900 is configured to be utilized in port sites within the body. For example, device 900 can be utilized with port sites including, but not limited to, catheter infusion sites, dialysis ports, and insulin ports, to treat and prevent infection. Such an embodiment can aid in ensuring the port or infusion site does not become infected or can be treated if it does get infected without actually removing the device that is inserted in the site. In some embodiments, output from device 900 is utilized after body piercing and/or tattooing to prevent or reduce the chance of infection. The device could be used for diagnostics or it could be used with a Smart Phone. The device 900 could be wireless with an endpiece that is operatively connected to a sensor that is implantable on the skin or in the body for sensing a body function or a fluid function, such as, chemistries, diabetes, blood sugar, oxygen tension, etc. Any implantable component could be partially biodegradable. Moreover, the device 900 or an associated end piece could be configured to use suction to adhere to the body to operatively position the sensors or hold the endpiece in position.

In one embodiment, device 100 may be configured to apply ultrasound topically or transdermally through small cannula with transducer 310 closer to the treatment site. In such an embodiment, there would be fluid inflow and fluid egress which would flush out the treatment area. During the flushing process, pharmaceuticals or other medicinal agent may be delivered locally or intravenously and the ultrasound would enhance the effect of the medicinal agent locally or in systemic pharmaceutical delivery. For example, stem cells may be delivered for chemotherapy. This could also be utilized for treatment or management of cancer. This could be used under image guidance. The ultrasound could be transmitted at the skin or through the skin to reach an implant or tissue. Characteristics of the ultrasound could be time varied based on the material property/thickness or biofilm materials could be separated. This could be done using two or more transducers at different locations.

As set forth above, applicator 104 may include imaging transducer 311. Ultrasound from imaging transducer 311 can be used purely diagnostically as the ultrasound may be able to have a different echogenic effect. For example, if there is biofilm attached to an implant, the ultrasound can detect the biofilm and device 100 would inform the surgeon the implant has an infection and should be treated in a different way. For example, an implant surface that is normal, without biofilm, would have one type of echogenicity, while an implant that has biofilm on it would have a different echogenicity because the biofilm would dampen the reflected signal received by imaging transducer 311. Thus, device 100 would inform the user that 1) the treatment site is infected and 2) biofilm treatment is required. The user then uses device 100 in the modality for treating biofilm so that biofilm can be removed from the implant, tissue or graft, without having to remove the implant. This may require one treatment or may require multiple treatments.

A specific area in which ultrasonic imaging and ultrasonic treatment of biofilm using device 100 is appropriate is total knee replacement. Typically, when there is an infection associated with a total knee replacement that lasts more than 2-4 weeks, there is an assumption that biofilm can grow on the implant and the entire implant needs to be removed.

However, in one example using device 100, one can leave the implant in position, treat the implant with ultrasound to remove the biofilm and all infectious agents, and then flush/irrigate the treatment site with antibiotics or other treatment agents. This procedure could be done either endoscopically or arthroscopically without having to remove the implant. For example, in diabetics, device 100 could be placed on a CPM (continuous passive motion device), which moves the joint through flexion and extension during treatment. The ultrasound may be positioned superficially and may be placed at different positions around the joint. Treatment time may be for several minutes in one location and then the position may be changed 90 degrees relative to an axis of the knee. The knee may be treated for several minutes at the second location and then rotated another 90 degrees. This may be repeated at, for example, 3-4 positions around the knee so that the biofilm could be treated around the entire circumference of the knee. This could also be done during endoscopic or arthroscopic surgery where ultrasound would be administered percutaneously, which allows the applicator 104 to be positioned closer to the implant in a fluent environment. With the applicator positioned in the percutaneous port, the joint would be moved through a range of motion so the ultrasonic vibration would help multiple surfaces of the implant to remove biofilm wherever it is attached to the implant and not simply in one location. Ultrasound delivery at the site of a joint replacement may require a placement of one ultrasonic transducer at a single location, or one or more transducers could be placed at 3 or 4 positions around the joint (e.g., circumferentially around the knee) for selected periods of time (e.g., 3, 5, or 10 minutes). A joint implant would be treated with one or multiple treatments to remove the biofilm. The treatment site may also be imaged using device 100 to determine if the treatment is effective and the biofilm is being removed. This procedure could also be used for stents, cardiac valves, grafts, bone grafts, tissue grafts, cages, etc.

Moreover, if bacteria is also adherent or if there is bacteria in the fluid of the joint, the fluid changes and its viscosity becomes thicker, sludgy, more opaque rather than simple bleeding, hematoma, or fluid around the joint which is usually clear yellowish fluid. Device 100 may be configured for determining if the fluid is denser or thicker and more viscous, indicating there is an infection. Ultrasound can be used to pick up more infectious agents and these echogenicity patterns could be cataloged and stored. If there is a difference in echogenicity of a fluid area, this would also be an indicator for infection and then device 100 may be configured to initiate treatments. For example, device 100 may be configured to be capable of changing the ultrasonic frequency from a first frequency associated with a diagnostic modality to a second frequency associated with a treatment modality to begin treatment of infection. As set forth above, the ultrasonic treatment may be pulsed or constant. It could be varied through several different frequencies to enhance the removal of biofilm. Since the tissue may be in a three-dimensional location, treatment with ultrasound may be made at varying angles. In the sinuses, for example, one location may be treated, but it may require multiple treatments at multiple locations. Each of the separate sinuses may need to be treated, and ultrasound may be applied at each different sinus location. Two ultrasounds or multiple ultrasounds may be applied to one location simultaneously or in a stable, staggered fashion. There may be two separate locations to stabilize and/or enhance the treatment.

As an example, a jig or fixture may be used on the surface of the body or within the body to ensure the various angles are reproducibly performed for a period of time. The applicator 104 is stabilized against the surface for a period of time and then moved to the next position so the three-dimensional construct is fully treated for biofilm. The jig (or simply landmarks) may be used to position and hold applicator 104 for necessary intervals of time whether it is 1 minute, 5 minutes, or 10 minutes. Treatment may need to be applied hourly or 2-3 times a day for 5-7 days for complete treatment. Device 100 or just applicator 104 can be incorporated with an external brace, an external jig or a fixator. Transducer(s) 310 could be implanted internally and then external energy could be applied to the internal transducer that is implanted. This would act as an energy directing device to direct the ultrasonic field to the specific location.

As disclosed above, in addition to bacterial infection, device 100 may be used for infections caused by virus, fungus, and/or mycoplasma, for example. Device 100 may also be used for chemotherapy for rapidly multiplying cells like tumor cells. With the tumor cells' ability to adhere, one could selectively modulate the frequency. When tumor cells grow very rapidly, they can become resistant to certain chemotherapy agents. Ultrasound and/or combination therapy as described herein may make these cancer cells more sensitive, especially if ultrasound is applied at a specific location and a specific depth.

Ultrasound can be applied in two or more specific locations, e.g., wrapped around the body to apply ultrasound to multiple specific locations in the body. Ultrasound can also be applied internally or percutaneously via a probe so it is closer to the affected site. For example, multiple probes may be offset 90 degrees to each other or a predetermined distance from one another (e.g., 1 centimeter) depending on drive frequency and location so that the probes can have a specific or more focused effect. This could also be done with ultrasound and/or resistive heating, pH sensitivity, pH deposits with pH releases, etc. This can be performed with pulsed electron therapy, such as cold plasma. This could be done in combination with ultrasound.

In one embodiment, device 100 may be used to treat a subject with an acute cardiac event (e.g., heart attack). During a heart attack, the cardiac vessels go into spasm and the blood sludges through the area of the heart. Using device 100, ultrasound can be applied to the heart area to cause vascular dilatation. The blood vessel would dilate rather than contract and spasm allowing relaxation of the blood vessel and then it could enhance the blood flow through the coronary artery or veins to decrease the risk of damage during a cardiac event (e.g., heart attack). Ultrasound from device 100 can also be used to treat deep vein thrombosis or pulmonary emboli. If a blood clot goes to the heart and then to the lungs, this area could clot off. If the area is acutely treated, the vascular spasm would relax and may enhance breaking up with or without thrombolytic treatments like Coumadin, Streptokinase, Lovenox, fractionated heparins, etc. One could relax the vessels to decrease spasticity and/or dilate the vessels to decrease the damage from the acute vascular event. Ultrasound could be used for peripheral vascular treatments or acute cardiovascular events. It could also be used for strokes. If a clot is present in the cranium of the skull, an implantable device could be used to percutaneously introduce ultrasound to treat the local area. If ultrasound is deliverable to the target area without bone, metal, or other artifact in the way, then one could directly treat the area using topical ultrasound application. If there is bone in the way or metallic device in the way, one may use percutaneous ultrasound using device 100 which could be inserted close to the target area. The ultrasonic energy could also be applied to treat spasticity to 1) dilate the vessel, 2) enhance blood flow, and/or 3) potentially breakup a clot. Vascular ultrasound could also be used with pulsatile treatments, other chemotherapeutic agents, or anti-thrombolytic treatments.

Device 100 could also be used as previously discussed to potentially prevent blood clots by keeping vessels and flow moving. This could be used topically around the extremities, for example, to prevent DVT after surgery or even during a surgical procedure. The ultrasound could be applied for periods of time, either constantly or intermittently, every hour or two, to a specific area targeting veins to decrease risks of deep vein thrombosis by dilating and causing fluid flow. This could be done in addition to pulsatile stockings or other thrombolytic agents. The device 100 can also be used to break up clots, knots, and mucus. It could liquefy such materials and improve fluid flow. This could be done in conjunction with suction or pressure. This could also be used in a stent with balloon dilatation via wire. The device 100 could be used for vascular treatments in a transcutaneous or a percutaneous manner.

It is also considered that a properly configured waveform could selectively disrupt or change the rate of the DNA to RNA to protein sequence. This could be used for therapeutic purposes, but also as an adjunct to diagnosis by allowing the technician to selectively modify the growth rate of certain specimens.

Although the device 100 has been described above as outputting a HFUS waveform optimized to treat infection in a body, device 100 can be configured to output a LFUS waveform optimized to treat infection in a body.

As discussed above, ultrasound, such as low frequency ultrasound or high frequency ultrasound, may be used to enhance delivery of a therapeutic agent through a bodily barrier, such as the skin, the blood brain barrier, a mucous membrane, etc. For example ultrasound having a frequency that is less than or equal to about 20 MHz may be topically applied to a bodily barrier along with the therapeutic agent. In some embodiments, the device 100 is configured to alternate between operating the transducer 310 in a low frequency mode and a high frequency mode to enhance delivery of the therapeutic agent. In still other embodiments, the device 100 includes a secondary acoustic transducer (not shown) configured to generate low frequency ultrasound, and the transducer 310 is configured to generate high frequency ultrasound at the same time or in an alternating fashion.

In general, it is thought that high frequency ultrasound can also be used to enhance delivery of a therapeutic agent through a bodily barrier such as an ocular barrier, a blood brain barrier, a mucous membrane, epithelial cells, a gut lining, skin, etc. Suitable ocular barriers include the corneal epithelial barrier and the blood-retinal barrier. High frequency ultrasound from the device 100 can be combined with the topical administration of a therapeutic agent to a body part at or adjacent one of these barriers to enhance penetration of the therapeutic agent through the barrier. Therapeutic ultrasound may also be combined with other treatment types, such as treatment by nebulizer, atomizer, netti pot, etc., to enhance therapeutic effect. As discussed above, the use of ultrasound to enhance penetration may be combined with any one or more additional penetration-enhancement modalities, such as lower frequency acoustic energy, ultraviolet light, vibration, etc., to improve delivery of the therapeutic agent.

High frequency ultrasound can also be used alone or in combination with another treatment modality to improve insulin levels in a diabetic patient. For example, in one or more embodiments, the device 100 is configured to deliver high frequency ultrasound directly to a patient's pancreas. For example, the device 100 can be configured to mount an array of ultrasound transducers 310 around the torso of the patient (e.g., the applicator 104 can include a belt that supports the transducers 310) to direct ultrasound to the pancreas. The transducers 310 can be configured to focus high intensity ultrasound at various focal points located throughout the pancreas. In other embodiments the transducers 310 can be surgically installed within the body of the patient, closer to the pancreas, to direct high frequency ultrasound directly to the pancreas. It is thought that high frequency ultrasound can stimulate insulin production in the pancreas. As an alternative to improving insulin levels by stimulating the pancreas, high frequency ultrasound can be used to increase vascular flow to improve the flow of insulin from an external insulin pump. As discussed above, high frequency ultrasound can improve vasodilation and other blood flow characteristics, which improve the flow of insulin to a patient from an external pump.

In addition to stimulating insulin production and flow, it is thought that high frequency ultrasound can be used to stimulate other bodily functions. For example, in one or more embodiments, the device 100 is used in a fertility treatment to stimulate the function of a reproductive organ and/or material. For example, the device 100 can be configured to deliver ultrasound to the ovaries of a patient to stimulate ovulation. It is thought that ultrasound can stimulate contraction of the ovaries, thereby promoting ovulation. The device 100 can be configured so that the transducer 310 is implanted at a location adjacent the ovary. The ultrasound can be focused at the ovary to avoid inadvertent stimulation of the uterus, which can cause disruption in ovulation. Certain frequencies and intensities of ultrasound are also thought to enhance sperm motility. Thus, the device 100 can be used to direct ultrasound to a patient's seminiferous tubules and/or epididymis to enhance the motility of sperm prior to ejaculation. Alternatively, the device 100 can be used to direct ultrasound to sperm that has already been ejaculated (e.g., sperm contained in a storage vessel such as a test tube or sperm traveling through a woman's reproductive anatomy) to enhance mobility. In addition to fertility treatments, the device 100 can be used to direct ultrasound toward stem cells to enhance stem cell response (e.g., stem cell division, tissue growth, etc.). It is believed that certain frequencies and intensities of ultrasound can stimulate obligatory asymmetric replication and stochastic differentiation of stem cells. It is also thought that certain frequencies and intensities of ultrasound can be used to promote tissue growth, such as bone growth and hair growth. Thus, in one or more embodiments, the device 100 is used to stimulate bone growth or hair growth by driving the transducers 310 to direct suitable ultrasound toward the desired bodily location. For example, the device 100 can be used to stimulate bone growth at the site of an implant to promote ingrowth.

It is also though that the device 100 can be used to treat pain. For example, when a patient presents with nerve injuries, neuralgia (e.g., postherpetic neuralgia, trigemninal neuralgia), bell's palsy, herpes zoster lesions, shingles, carpal tunnel, cubital tunnel, peripheral nerve entrapment, dermatitis, multiple sclerosis, migraine headaches, vasospastic reactions, etc., the device 100 may be used to treat the associated pain. For example, in one embodiment, the device 100 directs ultrasound energy toward or around the site of the pain to disrupt nervous system function there and thereby reduce pain. In another embodiment, ultrasound from the device 100 is combined with a topical pain medication. The device 100 can be configured to deliver ultrasound that enhances the permeability of the tissue and/or break down cell membranes at the location where the topical agent is applied. In some embodiments the device 100 is configured to alternate between modalities in which the ultrasound is configured to disrupt nervous function and promote penetration of topically administered therapeutic agents.

In addition to pain treatments, the ultrasound device 100 can be used to enhance delivery of therapeutic agents used to treat various diseases. For example, the device 100 can apply a suitable ultrasound to enhance the permeability of tissue in association with the topical treatment of psoriasis, basal cell cancer, melanoma, cellulitis, alopecia, Dupuytren's contracture, etc. The device 100 can be used with therapeutic agents that are delivered topically or in another manner, such as by injection. When the therapeutic agent is delivered by injection the ultrasound may preferably be focused at a focal region located at or adjacent to the depth of injection so that the delivery enhancements are achieved at the location where the therapeutic agent enters the tissue. The use of the ultrasound device 100 to increase the permeability of tissue and/or cells may also be combined with cosmetic treatments such as collagen enhancers, pigmentation (e.g., tattoos), lipophilic agents, Botox, hydrating agents, skin enhancers, etc. in various dermatological treatments, such as wrinkle treatments, skin discoloration treatment, treatment of spider veins, wart treatment, etc. In one embodiment, the ultrasonic device 100 is used in combination with sunscreen to enhance penetration into the skin and promote fuller coverage. Likewise, the device 100 can deliver ultrasound in combination with a suitable cream or ointment to treat topical reactions (e.g., allergic reactions, poison ivy, insect bites, parasites, hives, etc.), fungal infections, or nail bed lesions.

In some embodiments, pulsed or continuous ultrasound can be applied to the body to cause a topical therapeutic agent to penetrate the keratin layer of the skin. For example, the frequency of the ultrasound can be varied from about 15 kHz to about 3 MHz to cause the therapeutic agent to penetrate the keratin layer. In still other embodiments the ultrasound device 100 is configured to generate ultrasound that causes the therapeutic agent to penetrate through the epidermal layer into the dermal layer. These treatments can be used with or without solvents because the ultrasound enhances the permeability of the epidermal tissue sufficiently to allow penetration.

The ultrasound device 100 can also be used in invasive procedures. For example, the device 100 can apply ultrasound to a surgical site to enhance the local delivery of a therapeutic agent (e.g., an antibiotic) during surgery or needle guided radiological procedures. In the case of needle guided radiological procedures particularly, the ultrasound transducer 310 can be mounted on a needle or stylus. As discussed above, the ultrasound transducer 310 can also be mounted on a catheter. In these embodiments the ultrasound transducer is configured to be surgically positioned within the body of the patient at a normally inaccessible site. Thus, it is thought that the ultrasound transducer 310 could be configured to operate in a diagnostic mode before operating in a therapeutic mode. In the diagnostic mode, the transducer 310 may generate an ultrasound signal with a frequency of from about 1 MHz to about 10 MHz; and in the therapeutic mode, the transducer may generate an ultrasound signal with a frequency of from about 20 MHz to about 30 MHz. The use of diagnostic ultrasound could be combined with other diagnostic techniques (e.g., X-rays, MRI, PET scanners, etc.) to increase diagnostic certainty.

In one or more embodiments the device 100 is adapted for use in an oral therapy. For example, the device 100 may be adapted for use in oral pain management. Suitably, the applicator 104 can be configured to be operably mounted on the head of the patient to position the transducer 310 to direct ultrasound toward the treatment region. In one embodiment, the applicator 104 is shaped as a mouthpiece that is gripped between the teeth of the patient. In another embodiment, the applicator 104 is shaped as a face mask, with straps that support the face mask on the user's head. A mouth piece-shaped applicator 104 may, for example, position the transducer 310 inside the user's mouth. This configuration may be suitable for short treatment durations, on the order of a few seconds or minutes. The face mask may be well-suited for longer term treatments, such as overnight treatments, etc. Certain suitable oral therapies include the treatment of oral pain, oral infection treatment, etc.

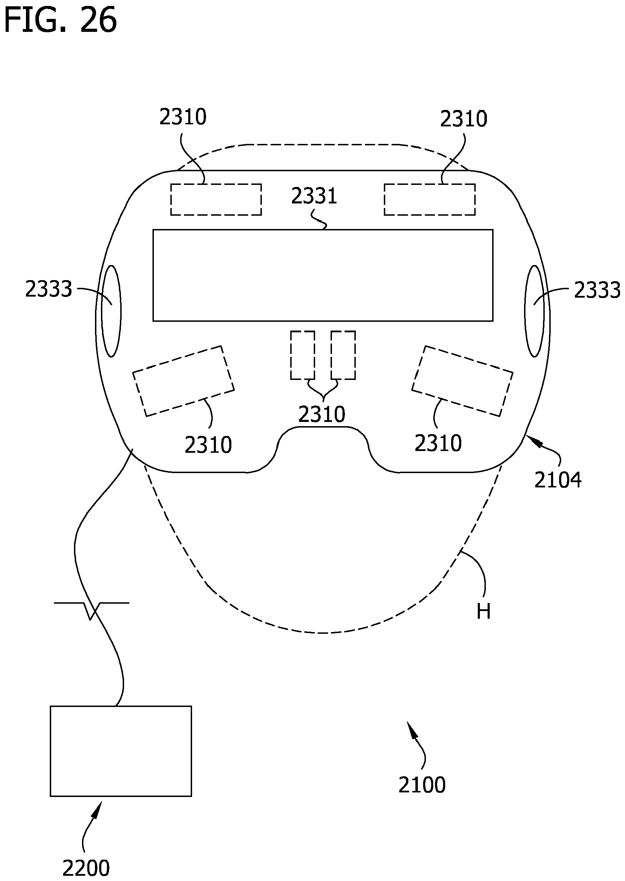

Referring to FIG. 26, another embodiment of an ultrasound device is generally indicated at 2100. Like the device 100, the device 2100 includes a control circuit 2200 that is operatively connected to an applicator 2104. In the illustrated embodiment, the applicator 2104 is a face mask that supports an array of ultrasound transducers 2310. When the face mask 2104 is worn on the head H of the user, the transducers 2310 are positioned generally over the sinus cavities of the user. The ultrasound transducers 2310 are operatively connected to a drive circuit of the control circuit 200 to receive energy that drives the transducers to transmit therapeutic ultrasound. As discussed above, the ultrasound energy can be used, for example, to treat a sinus infection in the user. Moreover, because the face mask 2104 includes an array of transducers positioned over all of the user's sinus cavities, any one or more (e.g., all) of the user's sinus cavities can be treated simultaneously or in sequence without repositioning the face mask.

The face mask 2104 preferably includes a built-in display 2331. The face mask 2104 is configured so that the display 2331 is positioned in front of the user's eyes during therapy. In one or more embodiments, the display 2331 is configured as an immersive display, such as a virtual reality display. Other conventional displays may also be used in other embodiments. In a suitable embodiment, the display 2331 is configured to display content that entertains and occupies the mind of the user during treatment. For example, in certain embodiments the control circuit 2200 can include a communications interface that selectively couples the device 2200 to a media device such as a smartphone or other internet device to receive media content from the media device. In the illustrated embodiment, the face mask 2104 also includes speakers 2333 (broadly, an audio transmission device) for transmitting sound to a user that is correlated with the display. In certain embodiments, the speakers 1331 and/or display 2331 are configured to present execution events associated with the treatment to the user. For example, when the device 2100 determines that the face mask 2104 is not properly installed, an audio indication can be provided to the user through the speakers 2333 and/or a visual indication can be provided through the display 2331.

Similar to the device 2100, the ultrasound device 100 can be configured to provide auditory stimulation to a user during treatment. In one embodiment, the device 100 can be connected to headphones or another audio transmission device to play music or another form of audible entertainment/relaxant. The communications interface 112 can connect the device 100 to a media content device such as a smartphone with media streaming capabilities or a media player with stored digital media content, etc. Thus, the device 100 can control the playing of media from the media content device to the user during treatment. In a suitable embodiment, the device 100 uses the auditory stimulation to provide an indication to the user of concurrent treatment events. For example, the device 100 can be configured to use the coupling determination step 710 of the method 700 (Fig. 9) to provide an indication about whether the applicator 104 is operatively connected to the user. Whenever the device 100 determines that the applicator 104 is operatively connected to the user, it provides auditory stimulation. But when the device 100 determines that the applicator 140 has become disconnected, it stops providing auditory stimulation to alert the user of the disconnection.

Instead of configuring the ultrasound device 100 to be connected with a separate audio transmission device, the device can be configured to modulate an acoustic signal in the audible range onto the ultrasound. Suitably, the control circuit 200 can modulate the auditory signal onto the ultrasound such that the auditory signal is self-demodulated as the signal passes into tissue. For example, if the ultrasound is applied at the head of the user, the auditory signal is demodulated as the signal enters the skull, allowing the user to hear the content of the auditory signal.

The ultrasound device 100 can be configured for use with a single user or multiple users. In some embodiments, the ultrasound device 100 includes a transmission component that is custom molded to contour to a body portion of a specific user. In other embodiments, the transmission component 330 can be formed to contour to the general shape of a particular body portion, so that the device can be used to transmit ultrasound to that body portion on many different users. The ultrasound device 100 can also be configured for reuse with different users. As discussed above, the transmission component 330 of the applicator 104 is configured for contact with the user. If the device 100 is to be reused for different users, the contact component 330 is preferably covered or sterilized between uses. In one embodiment, the applicator is fully moisture sealed so that it can be disconnected from the device 100 and sterilized in an autoclave. In another embodiment, the transmission component 330 can be selectively removed from and reinstalled on the applicator 104 to allow for sterilization of the transmission component in an autoclave. In still other embodiments, the applicator 104 is sized and arranged for mounting a disposable sterile cover that fits over the transmission component (not shown). The disposable sterile cover is configured to provide acoustic coupling between the transmission component 330 and the body of the user.

It is believed that the therapeutic effect of the ultrasound can be improved, in some instances, by controlling the pH of the treated region of the body. Thus, in certain embodiments, a buffer, a phenol, an alcohol, or another solvent such as a surfactant or a DMSO is topically applied to the treatment region before, during, or after the ultrasound is administered. These fluids can be used to affect the pH of bodily tissue. In other embodiments, optical therapies, such as ultraviolet light therapy or laser therapy, are combined with the ultrasound to control environmental parameters such as pH. Magnetic therapies and electrical therapies (e.g., radio frequency therapies, electroshock therapy, etc.) may, likewise, be combined with ultrasound therapy and/or other therapies such as drug therapies, to improve the effects of such therapies and/or provide pain relief.

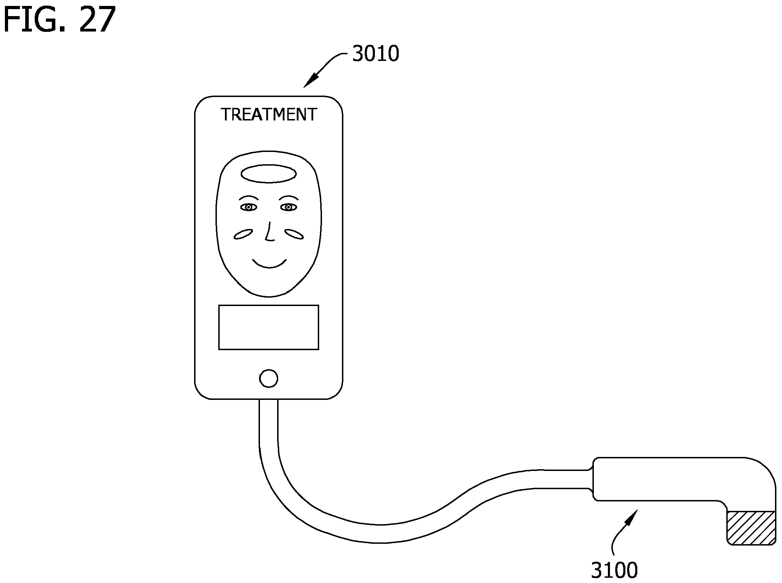

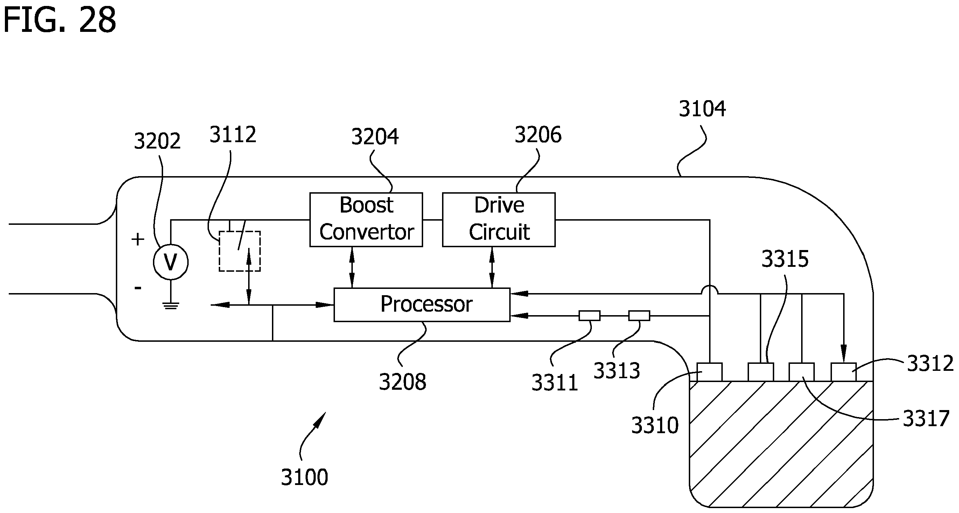

Referring to Figs. 27 and 28, an ultrasound device configured for use with a mobile device 3010, such as a smartphone or tablet, is generally indicated at 3100. As explained below, the smartphone-connected ultrasound device 3100 allows for enhanced compliance safeguards and user feedback in ultrasound treatment. The control circuit of the device 3100 is fully contained within an applicator housing 3104. In the illustrated embodiment, the device 3100 includes a battery 3202 that powers the device. The battery 3202 may suitably be a rechargeable battery that is selectively connectable to a charge circuit. The battery 3202 is connected to a boost circuit 3204 to provide power to the boost circuit. The boost circuit 3204 is operatively connected to a drive circuit 3206 and configured to amplify the DC voltage it receives from the battery 3202 and provide an amplified DC voltage to the drive circuit. The drive circuit 3206 powers the ultrasonic transducer 3310, which is positioned at the distal end of the applicator housing 3104. A contact component 3330 is mounted on the applicator housing 3104 and is operatively connected to the ultrasound transducer 3310 to operatively couple the transducer to the user.

The device 3100 includes a processor 3208 that is operatively connected to the boost converter 3204 and drive circuit 3206 to control the delivery of power to the ultrasonic transducer 3310. The processor 3208 is also connected to a voltage monitor 3311 and current monitor 3313 that provide the processor with feedback information about the voltage and current supplied to the transducer 3210. In addition, the processor is connected to a temperature sensor 3315 and a capacitance sensor 3317 that are configured to sense the temperature and capacitance at the contact component 3330. The processor 3208 is further coupled to a vibrator 3312 that is configured to provide tactile feedback to the user about treatment events. The vibrator 3312 suitably receives power from the power supply 3202. A communications interface 3112 allows the processor 3208 to communicate with the mobile device 3010. In certain embodiments, the communications interface 3112 is a hardwire communications port (e.g., a USB port), and in other embodiments the communications interface facilitates wireless connection between the processor and a mobile device.

In the illustrated embodiment, the mobile device 3010 is configured to run an application that controls and monitors the delivery of ultrasound to the user. For example, the application can provide a user interface through which the user can select one from a catalog of treatment regimens. The mobile device 3010 can either locally store or have web-based access to the catalog of treatment regimens for various ultrasound therapies. The user interface provided by the application can initially present an overarching view of a human body. By selecting an active portion of the body graphic, the user can drill down to a more specific body part. For example, when the user selects the head of the body, the application navigates to another view that presents a graphic representation of the head. This more detailed view can include selection objects for navigating to further magnified views and/or selection objects for selecting particular treatment regimens associated with the head of a user.

When the user selects a treatment regimen, the mobile device 3010 provides suitable control instructions to the processor 3208 of the ultrasound device 3100 to run the selected treatment regimen. The mobile device 3010 can provide real time control of the processor 3208, or it can download all or some of a control routine to the device 3100 so the processor runs the routine independently of further input from the mobile device. In suitable embodiments, the processor 3208 receives data from the capacitance and temperature sensors 3315, 3317 that either the processor or the mobile device 3010 uses to determine when the contact component 3330 is operatively connected to the correct body portion of the user. When temperature and capacitance readings are within acceptable ranges for the selected body portion, the processor 3208 or mobile device 3010 determine that the contact component 3330 is operatively connected to the selected body portion.

After determining the device 3100 is operatively connected to the selected body portion, the processor 3208 can then operate the vibrator 3312 to provide a tactile indication to the user that the applicator housing 3104 is properly positioned and/or that ultrasound treatment is about to begin. With the contact component 3330 operatively connected to the user, the processor 3208 causes the boost converter 3204 and drive circuit 3206 to deliver power to the transducer 3310 to generate ultrasound at the frequency and intensity associated with the selected treatment regimen. During ultrasound transmission, the processor 3208 continues to receive feedback from the temperature and capacitance sensors 3315, 3317, as well as the current and voltage sensors 3311, 3313. If the processor 3208 or mobile device 3010 determines, based on this feedback, that the contact component 3330 has shifted out of position, the processor causes the vibrator 3312 to provide a tactile indication to the user. If the user does not reposition the applicator 3104 within a predetermined amount of time, the processor discontinues ultrasound transmission until the user repositions the contact component 3330 in operative connection with the selected body region. The processor 3208 continues to operate the boost converter 3204 and drive circuit 3206 until the treatment regimen is complete. Upon completion, the processor 3208 causes the vibrator 3312 to provide a tactile indication to the user.

Throughout the treatment regimen, the processor 3208 provides data to the mobile device 3010 that can be used to provide user feedback and compliance monitoring. For example, the processor 3208 suitably provides information to the mobile device 3010 about the operation of the boost converter 3204 and drive circuit 3206, as well as the feedback signals from the current and voltage monitors 3311, 3313 and temperature and capacitance sensors 3315, 3317. The application on the mobile device 3010 is configured to analyze the data it receives from the processor 3208 to provide feedback to the user about the treatment. Suitably, the mobile device 3010 stores data from multiple treatment sessions that can be presented to the user for tracking progress over time. Moreover, in some embodiments, the mobile device 3010 communicates with a central database of a compliance monitoring system that stores user information, such as treatment plans set forth by a medical provider. After each treatment, the mobile device 3010 communicates the results of the treatment to the compliance monitoring system, which can provide notifications to the medical provider about whether or not the user is complying with the treatment plans. Thus, the mobile device 3010 can be used to aid a medical provider in ensuring compliance with treatment expectations and evaluating the effectiveness of treatment based on the actual therapies delivered to the user.

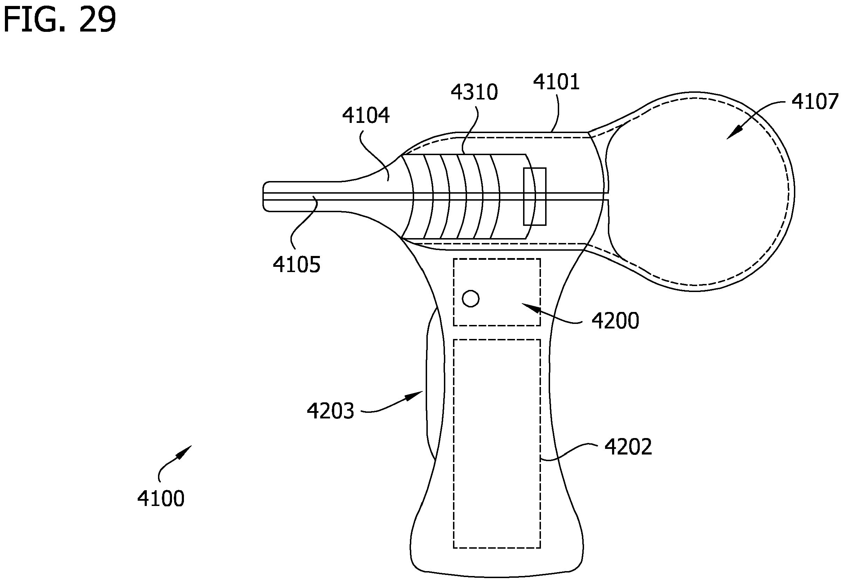

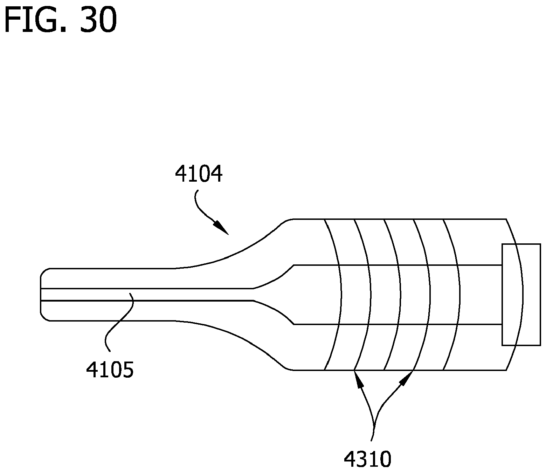

Referring to Figs. 29 and 30, another embodiment of an ultrasound device is generally indicated at 4100. As will be explained below, the ultrasound device 4100 is particularly suited for treatment of otitis media (e.g., ear infections). The device 4100 includes a housing 4101 that can be sealed against the ingress of liquids. The housing 4101 supports a control circuit 4200 (including, for example, a drive circuit as discussed above), a power supply 4202 (e.g., a battery), and an ultrasound transducer 4310. These features can be adapted to function similar to the control circuit 200, the power supply 202, and the transducer 310 discussed above. In certain embodiments the housing 4101 can include separable modules for electronics and application components that are electrically connectable. The control circuit 4200 is operatively connected between the power supply 4202 and transducer 4310 to cause the transducer to generate therapeutic ultrasound. A trigger 4203 is operatively connected to the control circuit 4200 to selectively actuate the device.

The housing 4101 includes a cannulated applicator nozzle 4104 that is sized and arranged for being received in the outer ear of a user. The nozzle 4104 defines a lumen 4105, and the transducer 4310 is operatively connected to the nozzle to propagate ultrasound through the lumen. The housing 4101 further supports a fluid reservoir 4107 configured to contain an ultrasound coupling fluid. The fluid reservoir 4107 is operatively connected to the lumen 4105 to deliver the coupling fluid to the lumen. In the illustrated embodiment, the reservoir 4107 is positioned proximally of the ultrasound transducer 4310 and delivers fluid through a flow passage that extends through a hole in the transducer. But in other embodiments, the device 4100 can have other arrangements.

The applicator nozzle 4104 is configured to be received in the outer ear of a user. Although the illustrated applicator nozzle is sized and shaped for being received in a user's ear, it will be understood that applicator nozzles that are sized and shaped for being received in other body orifices may also be used without departing from the scope of the invention. Examples of other suitable body orifices include a urethra, a vagina, an anus, a nostril, a mouth, a surgical port connected to a body part such as a kidney, a bladder, a prostate, a pancreas, etc.

In use, the distal end portion of the applicator nozzle 4104 is operatively inserted into the outer ear of the user. With the device 4100 in proper position, the user can depress the trigger 4203 to activate the device. In a suitable embodiment, the control circuit 4200 includes a controller and a feedback device for automatically controlling treatment once the trigger 4203 is depressed. The controller first causes fluid to be dispensed from the reservoir 4107 (e.g., using a pump or other flow device) through the hole in the ultrasonic transducer 4310 and the lumen 4105 in the nozzle 4104. The fluid passes through the device 4100 and fills the ear canal. The control circuit 4200 can include sensors that determine when the fluid fills the ear canal sufficiently to operatively couple the ultrasound transducer 4310 with the tympanic membrane of the ear. For example, the sensors can detect a back pressure in the lumen 4105 and hole in the ultrasonic transducer 4310, which indicates fluid connection between the transducer and tympanic membrane created by the coupling fluid. Once the controller determines that the transducer 4310 is operatively coupled with the tympanic membrane, it causes the transducer to generate therapeutic ultrasound

The ultrasound is transmitted through the coupling fluid to the tympanic membrane, causing vibration of the tympanic membrane. The vibration of the tympanic membrane is believed to cause drainage of the Eustachian tubes into the pharynx. This occurs without the fluid penetrating the tympanic membrane. In one embodiment, the controller is configured to apply the therapeutic ultrasound for a period of from about 3 minutes to about 5 minutes before automatically shutting off and providing the user an indication of the end of treatment (e.g., a vibration, a sound, etc.). It is thought that the use of ultrasound alone could reduce pressure by liquefying material in the Eustachian tubes a sufficient amount to cause drainage. In addition, it is thought that the ultrasound itself could treat the bacteria that is causing infection, such as by degrading biofilm bacteria to a planktonic state. It will be understood that the controller could be configured to alternate between different treatment modalities that improve drainage and affect bacteria. Furthermore, one or both of these treatment methods could be combined with other therapies, such as antibiotics, irrigation, etc. For example, in one or more embodiments, the ultrasound coupling fluid in the reservoir 4107 includes a therapeutic agent, such as an antibiotic, for treating infection.

Although the use of the device 4100 was discussed in reference to the treatment of an ear infection, it will be understood that other embodiments can be used to treat other body parts. For example, the device could be used to treat various infections of the body by positioning the nozzle in an orifice and operatively connecting the ultrasound transducer with the infected body portion by delivering ultrasonic coupling fluid to the infected body portion. Likewise, the device could be used to break up occlusive or low flowing slugs of material by inserting the applicator in an orifice and operatively connecting the ultrasound transducer with the slug by delivering fluid to a location in the body that allows ultrasound from the transducer to cause vibration of the slug.

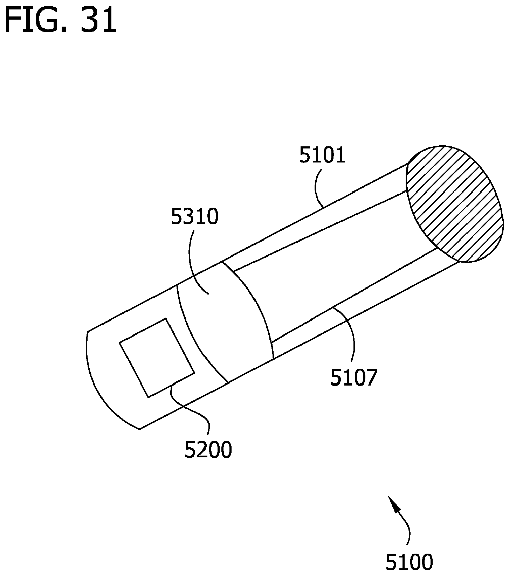

Referring to Fig. 31, another embodiment of an ultrasound device is generally indicated at 5100. As will be explained below, the ultrasound device 5100 is suitable for applying a topical therapeutic agent. For example, in certain embodiments, the device 5100 is suitable for applying an ultraviolet light protection agent, a moisturizer, a balm, a lotion, an ointment, etc. to the skin or lips of a user. It is contemplated that the device 5100 can be constructed as a disposable, one time use device. The device 5100 can also be refillable and reusable.

The device 5100 includes a housing 5101 that supports control circuit 5200, an ultrasound transducer 5310, and a support 5107 for holding a therapeutic agent. The control circuit 5200 suitably includes a power supply (not shown) that is configured to supply power to the ultrasound transducer 5310. The control circuit 5200 can also include a switch (not shown) that the user can actuate to activate the device 5100. In certain embodiments, the control circuit 5200 is constructed for low cost, by simply allowing the user to switch the ultrasound transducer 5310 between operating and non-operating modes. In other embodiments, the switch can provide the capability for the user to select one of a plurality of ultrasound frequencies and/or intensities, thus allowing the user to select between multiple treatment modalities. In still other embodiments, the control circuit 5200 includes a controller that automatically controls the transducer 5310 after the device 5100 is switched on. The therapeutic agent can suitably be a solid or solid-like material or a fluid. Preferably, the therapeutic agent functions both for therapeutic effect and as an ultrasound coupling material.

In one embodiment, the support 5107 is a platform that supports a waxy therapeutic agent such as a balm or a cosmetic agent for application on the lips or skin. The waxy body of therapeutic agent can be selectively extended through an opening in the distal end of the housing 5101 to allow the therapeutic agent to be applied directly to the desired body part of the user. In one embodiment, the material is extended and retracted by selectively moving the platform 5107 relative to the housing 5101 (e.g., using a screw mechanism, not shown). The transducer 5310 can suitably be configured to move with the platform 5107 relative to the housing 5101 to remain in operative connection with the therapeutic agent, regardless of the position of the platform.

In other embodiments, the support 5107 is a reservoir for holding a liquid therapeutic agent, such as a viscous ointment or lotion. In certain embodiments, the fluid is dispensed through an outlet aperture in the distal end of the housing 5101 by driving a plunger (not shown) distally through the reservoir 5107 (e.g., using a screw mechanism, not shown). The transducer 5310 can suitably be configured to move with the plunger relative to the housing 5101 to remain in operative connection with the therapeutic agent, regardless of the position of the plunger.

In use, the user switches on the device 5100 and applies the therapeutic agent to the skin. The ultrasound transducer 5310 transmits ultrasound that propagates through the reservoir 5107 or waxy body of therapeutic agent to the treatment site. In a suitable embodiment, the transducer generates ultrasound that has a frequency of less than about 20 MHz. This causes the ultrasound to pass into the skin or lips of the user and enhance treatment by, for example, enhancing delivery of the therapeutic agent.

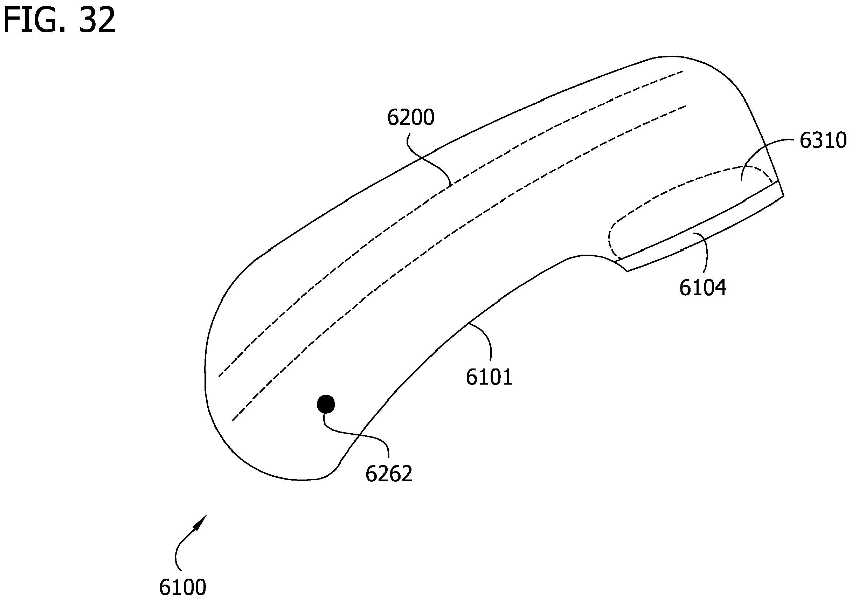

Referring to Fig. 32, another embodiment of an ultrasound device is generally indicated at 6100. The ultrasound device 6100 includes a housing 6101 that supports a control circuit 6200, including a power supply 6202. The control circuit 6200 is operatively connected to a transducer 6310 configured to generate therapeutic ultrasound. In suitable embodiments, the control circuit can include a switch or other actuator that the user can utilize to selectively activate the device 6100. The control circuit 6200 can have any of the control and drive features discussed above in reference to other embodiments of ultrasound devices. In certain embodiments, the transducer 6310 is configured to generate ultrasound having a frequency of from about 10kHz to about 1 MHz and an intensity of less than about 0.125 W/cm$^2$.

The housing 6101 is configured to hold a disposable preloaded therapeutic agent applicator 6104. The applicator 6104 functions to both hold a topical therapeutic agent and apply the therapeutic agent when placed in contact with the user. In suitable embodiments, the applicator 6104 is a pad or other structure made of foam or fabric (e.g., gauze, etc.) that is impregnated with the therapeutic agent. Multiple applicators 6104 may be packaged together in moisture proof packaging so that individual applicators can be removed from the packaging, used, and disposed of. In certain embodiments, the applicators 6104 are impregnated with topical therapeutic agents for application on the skin of a user. For example, the applicators 6104 can be saturated with a face wash, an exfoliator, a moisturizer, a rash treatment, a wrinkle reducing agent, or another skin enhancer, among other possible therapeutic agents. Suitably, the therapeutic agent or material that holds the therapeutic agent functions as an ultrasound coupler for operatively coupling the transducer 6310 with the skin of the user (or other body part) during treatment.

To use the device 6100, the user removes an applicator 6104 from its moisture resistant packaging and installs it on the housing 6101. When the applicator 6104 is properly installed, it has an exposed surface at the distal end of the housing 6101. The user switches on the device 6100 and the control circuit 6200 directs power from the power supply 6202 to the transducer 6310. The user places the applicator 6104 in contact with the skin or other body part, thereby delivering the therapeutic agent to the treatment area. The transducer 6310 generates ultrasound that travels through the applicator 6104 to the user. The ultrasound simultaneously increases the permeability of the treatment area and drives the therapeutic agent into the treatment area, thus enhancing the effectiveness of the therapeutic agent.

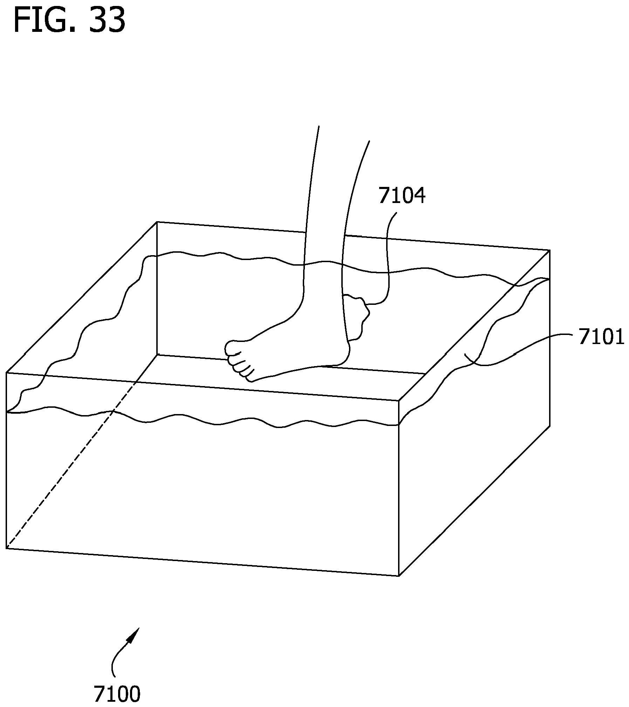

Referring to Fig. 33, another embodiment of an ultrasound device is generally indicated at 7100. The ultrasound device 7100 includes a reservoir 7101 for containing a bath of water or another fluid for immersing a portion of a user's body. Suitably, the fluid can function as an ultrasound coupler. In addition, the fluid can include one or more therapeutic agents for treating a condition of the user. An ultrasound applicator 7104 that includes at least one ultrasound transducer (not shown) can be positioned in the bath to deliver ultrasound to the user. The transducer(s) may be configured to deliver ultrasound having a frequency of from about 10 kHz to about 15 MHz and an intensity of from about 0.1 W/cm$^2$ to about 3 W/cm$^2$. In suitable embodiments, the applicator 7104 is a waterproof device that can be moved to a desired position within the reservoir 7101 for operative connection with the user. In other embodiments, the applicator 7104 includes an array of transducers that are fixed to the walls 7101 of the reservoir to generate ultrasound that permeates the bath.

In use, the applicator 7104 can apply ultrasound to a treatment area before, during, or after the body part is positioned in the bath 7101. In a suitable method of using the device 7100, the user positions the body part in the bath 7101 while the applicator 7104 delivers ultrasound through the bath to the treatment area. It is contemplated that the ultrasound device may be particularly well-suited for treating wounds such as burns, sores, skin breakdowns, and diabetic ulcers. In certain embodiments, the device 7100 is configured to deliver ultrasound to the user in multiple modalities. For example, during a first period of time the applicator 7104 can deliver a therapeutic ultrasound to the user that is suitable for wound debriding. During a second period of time the applicator 7104 can deliver a therapeutic ultrasound that enhances the delivery of a therapeutic agent to the user (e.g., using sonophoresis).

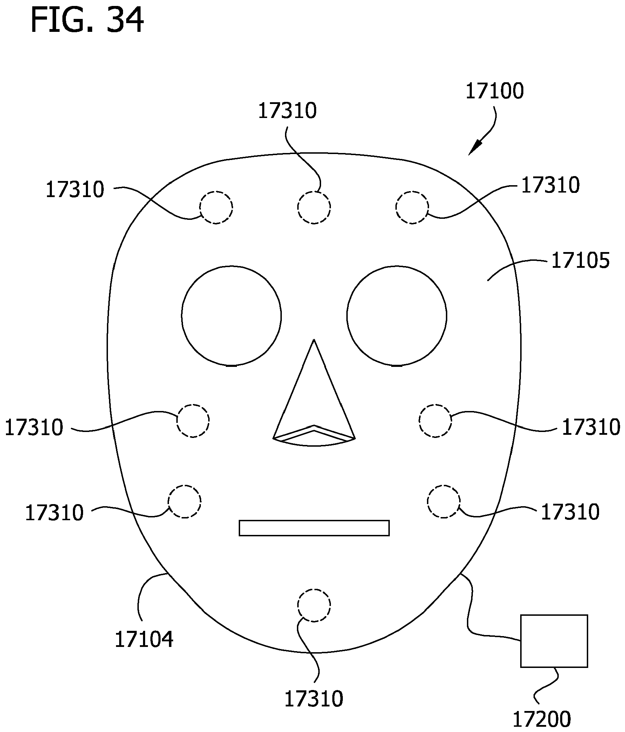

Referring to Fig. 34, another embodiment of an ultrasound device is generally indicated at 17100. The ultrasound device 17100 includes a control circuit 17200 that is operatively connected to an applicator 7104 suitable for being positioned over the face of a user. The control circuit 17200 can have any of the features of the control circuits of other ultrasound devices discussed above. But preferably, the control circuit 17200 is configured to power ultrasound transducers 17310 that are mounted on the applicator 17104. In one or more embodiments, the transducers 17310 are configured to generate therapeutic ultrasound having a frequency of from about 10 kHz to about 15 MHz with an intensity of from about 0.1 W/cm$^2$ to about 3 W/cm$^2$.

The applicator 17104 includes a face mask support 17105 that is sized and shaped for covering at least a portion of the face of the user. In the illustrated embodiment, the face mask support 17105 is configured to contact and cover the forehead, cheeks, chin, and nose of the user and includes holes for positioning over the eyes, mouth and nostrils. Suitably, the face mask support 17105 can be a gel pad that is conformable to the shape of the user's face and functions as an ultrasound coupler. The ultrasound transducers 17310 are mounted on the support 17105 such that the gel pad operatively couples the transducers with the user's face. In one or more embodiments, the face mask support 17105 includes a distal layer that is configured to receive a therapeutic agent that the applicator 17104 positions in contact with the user's face during use. In suitable embodiments, the gel pad is configured to gradually release the therapeutic agent over a predetermined time period (e.g., 24 hours, etc.). In certain embodiments, the gel pad includes adhesives that hold the support 7105 on the user's face. In other embodiments, the support 17105 includes straps that mount the applicator 17104 on the head of the user.

In use, the control circuit 17200 controls the delivery of ultrasound to the face of the user. The control circuit 17200 powers the transducers 17310 and causes them to selectively deliver ultrasound during the period of release of the therapeutic agent from the gel pad. Suitably, the gel pad 17105 is configured to retain the therapeutic agent during the treatment period. Thus, using the gel pad prevents loss of therapeutic agent during treatment. Although the illustrated gel pad 17105 is shaped for application on a user's face, other gel pads can be used for other body parts. For example, in one embodiment, the gel pad or other slow release applicator is shaped and arranged for covering all or part of a user's leg. It is thought that such an applicator may be used in treatment of restless leg syndrome.

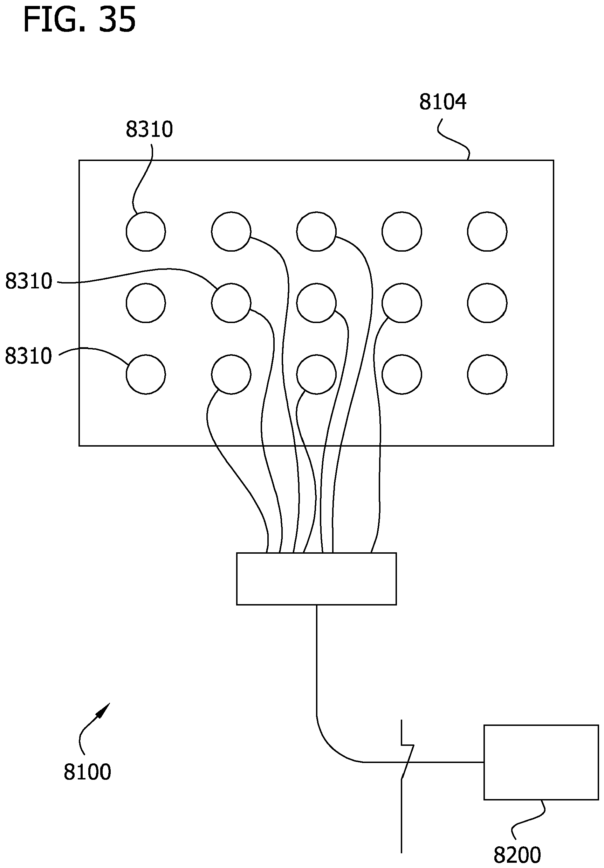

Referring to Fig. 35, another embodiment of an ultrasound device is generally indicated at 8100. The ultrasound device 8100 includes a control circuit 8200 that is operatively connected to an applicator patch 8104 suitable for being positioned on a treatment area of a user. The control circuit 8200 can have any of the features of the control circuits of other ultrasound devices discussed above. But preferably, the control circuit 8200 is configured to power an array of ultrasound transducers 8310 that are mounted on the applicator patch 8104. Suitably, the applicator patch 8104 can include adhesive for mounting the patch on a body portion of a user so that the transducers 8310 are positioned over the treatment area. In certain embodiments, the applicator patch 8104 is fastened to the user with an adhesive gel that also functions as an ultrasound coupler. In one or more embodiments, the transducers 9310 are configured to generate therapeutic ultrasound having a frequency of from about 10 kHz to about 15 MHz with an intensity of from about 0.1 W/cm$^2$ to about 3 W/cm$^2$.

In certain embodiments, the applicator patch 8104 is configured so that the user can selectively couple specified ones of the transducers 8310 to the control circuit 8200. For example, in the illustrated embodiment, the user individually selects transducers 8310 by hardwiring the selected transducers to the control circuit 8200. Thus, the patch 8104 may include wiring ports for each of the transducers 8310. In other embodiments, the patch 8104 includes a single port for connecting the transducers 8310 to the control circuit module 8200 and one or more switches for selecting those of the transducers that are to generate ultrasound. In still other embodiments, the patch 8104 is configured so that the user can select transducers by cutting the patch to conform in shape with the treatment area. Only the transducers 8310 that remain after cutting are then connected to the control circuit 8200.

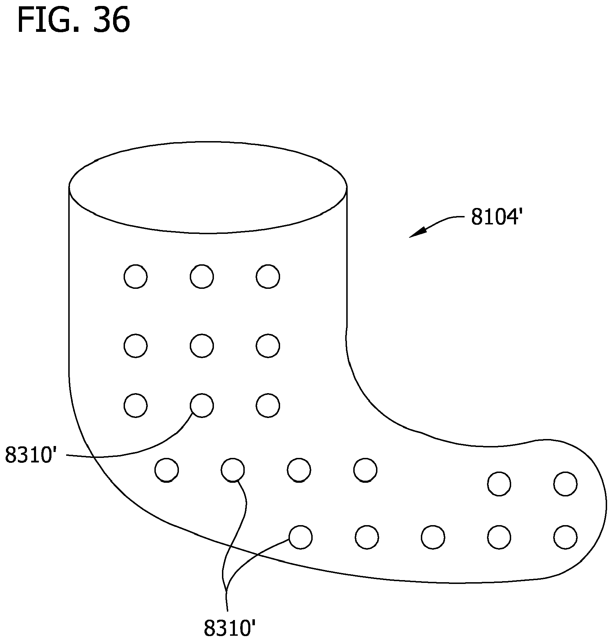

It is contemplated that the device 8100 is particularly well suited for wound and/or tissue necrosis treatments. It will be understood that the ultrasound may be used in combination with a therapeutic agent to treat pain or infection at the wound site. In addition, the application of energy to the selected transducers 8310 can be alternated so that the location at which ultrasound treatment is being applied changes over time. Because the chosen transducers 8310 are selected by the user, the patch can be customized for targeted treatment of a particular wound site. Moreover, because an array of transducers 8310, rather than a single transducer, is used, the ultrasound is spread out for full coverage of the wound site. It will be understood that the concept of arranging the transducers of an applicator in an array can be used in other contexts. For example, an array of transducers may be installed in an insole for being received in a shoe. Such an embodiment may be useful, for example, for treating diabetic ulcers on a user's foot. In addition, as shown in Fig. 36, an array of transducers 8310' can be mounted on a stocking 8104' or other undergarment.

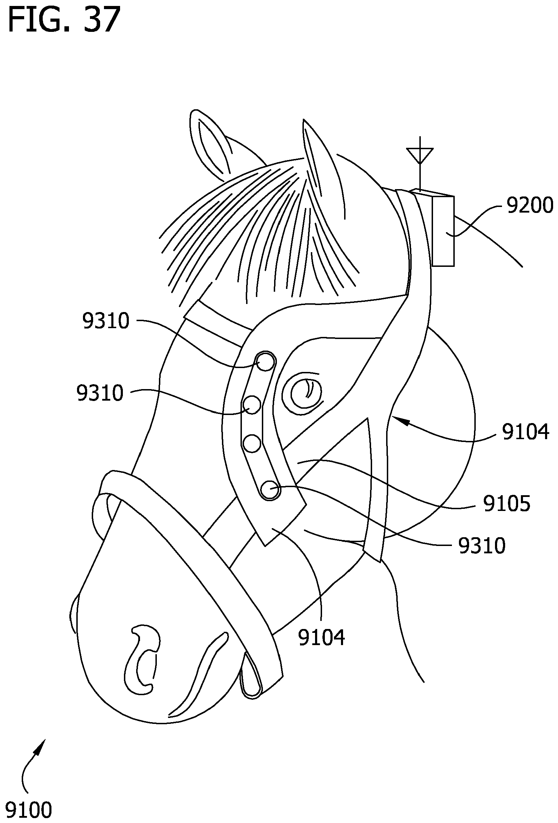

Referring to Fig. 37, in one or more embodiments, an ultrasound device, generally indicated at 9100, is suitable for use in veterinary applications. The ultrasound device 9100 includes a control circuit 9200 that is operatively connected to an applicator 9104 suitable for being positioned over the head of a horse to direct therapeutic ultrasound to the sinus cavities of the horse. Thus, as explained below, the ultrasound device 9100 is configured for treating a sinus infection or a clogged sinus duct in a horse. It will be understood that the device 9100 could also be adapted to treat other infections and conditions in other animals. The control circuit 9200 can have any of the features of the control circuit of other ultrasound devices discussed above. But preferably, the control circuit 9200 is configured to power the ultrasound transducers 9310 that are mounted on the applicator 9104. In one or more embodiments, the transducers 9310 are configured to generate therapeutic ultrasound having a frequency of from about 10 kHz to about 15 MHz with an intensity of from about 0.1 W/cm$^2$ to about 3 W/cm$^2$. In certain suitable embodiments, the control circuit 9200 is configured to communicate with a mobile device, as discussed above in reference to Figs. 27-28.

The applicator 9104 includes a bridle 9105 that mounts the ultrasound transducers 9310 over the sinus cavities of the horse (e.g., one or more transducers over the frontal sinus, one or more transducers over the caudal maxillary sinus, and/or one or more transducers over the rostral maxillary sinus). In addition, the bridle 9105 can support one or more feedback devices (e.g., temperature sensors, etc.) that provide feedback information to the control circuit 9200 about the operation of the device 9100. During use of the device 9100, the control circuit 9200 delivers power to the transducers 9310, which causes the transducers to deliver therapeutic ultrasound to the sinus cavities of the horse. The ultrasound is thought to break apart blockages and remove biofilm bacteria that causes infection.

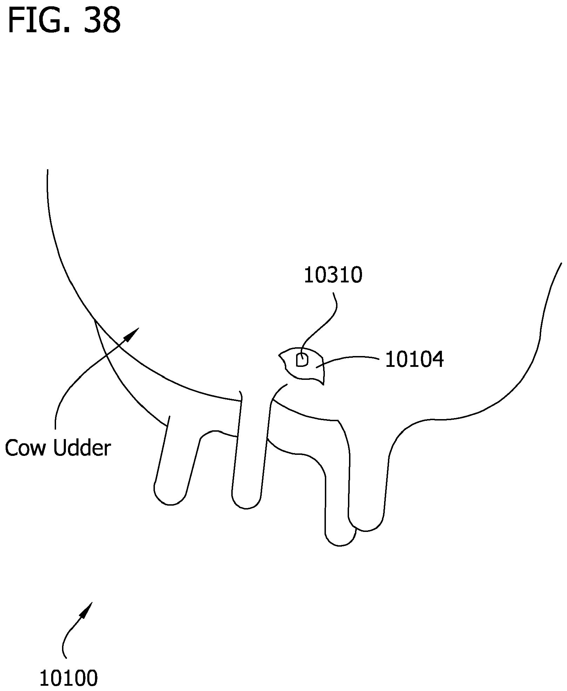

Referring to Fig. 38, in another embodiment, an ultrasound device generally indicated at 10100 is configured for treatment of mastitis or clogged lactation ducts. Although the illustrated embodiment is shown in use with a cow utter, it will be understood that the device 10100 could be adapted for use in treating the lactation ducts of other mammals (e.g., humans) in other embodiments. The device 10100 includes an applicator patch 10104 that can be fastened (e.g., using adhesives) to an utter of a cow over a clogged duct or a site of mastitis. The applicator patch 10104 includes an ultrasound transducer 10310 configured to generate a therapeutic ultrasound for breaking up the material that is clogging the duct and/or treating the infection that is causing mastitis. As will be understood, the patch 10104 can be selectively connected to a control circuit (not shown), which can suitably be configured to control the ultrasound transducer 10310. In certain embodiments the patch 10104 supports ultrasonic coupling gel that contains a therapeutic agent for treating infection. Thus, in addition to using the ultrasound to treat infection by removing biofilm, the ultrasound can be configured to enhance delivery of a therapeutic agent to the site of infection.

It is contemplated that ultrasound devices can be adapted for various additional veterinary applications other than those described in detail above. For example, in the case of a torn muscle in an animal, ultrasound may be applied, with or without an additional therapeutic agent, to stimulate tissue healing and regrowth. It is thought that the use of ultrasound in veterinary treatment may be particularly well-suited to large animals, but ultrasound may also be used to treat smaller animals, such as companion animals, in other embodiments.

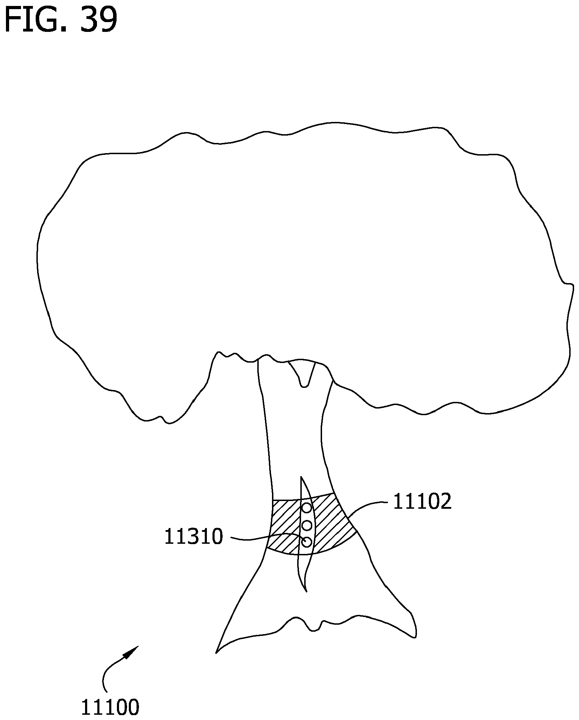

In addition to the use of ultrasound for treating animals, it is thought that ultrasound therapy can also be used in botany. For example, in some instances, storms or other events can cause damage to plant tissue. For example, as shown in Fig. 39, the trunk of a tree may become damaged. An ultrasound device generally indicated at 11100 is configured to be mounted on the tree to treat the damage. The ultrasound device 11100 includes a strap 11102 configured to mount the device on the tree and an ultrasound transducer 11310 that is positioned over the site of damage when the strap is installed on the tree. The transducer 11310 is configured to receive power from a control circuit (not shown), which can have any of the features of the control circuits used in other ultrasound devices discussed above. In use, the device 11100 delivers therapeutic ultrasound to the tree over the site of the damage to stimulate growth of the plant tissue and repair of the damage. The use of ultrasound can be combined with a therapeutic agent that promotes plant tissue growth, or it could be used alone in other embodiments.

In another embodiment, the ultrasound device 11100 can be configured to apply ultrasound to the roots of a plant. For example, the ultrasound device may include a ring that is configured to extend circumferentially around the base of the plant as it is supported on the ground. The device can include an array of ultrasound transducers that direct ultrasound through the ground and into the roots. It is thought that the applied ultrasound can stimulate the roots to absorb more water and other nutrients. Thus, the ultrasound may be applied before, after, or during the delivery of a nutrient supplement to the roots. It is contemplated ultrasound may be applied to a plant that is rooted in the ground or in a pot.

Referring again to Fig. 3, the treatment applicator 104 includes the vibratory device 312, which is configured to selectively provide tactile or haptic feedback to a subject during treatment. In one or more embodiments, the applicator 104 is configured to activate the vibratory device 312 whenever the ultrasonic transducer is active to provide the subject and/or treatment administrator with a tactile or haptic feedback that treatment is active. Alternatively, tactile or haptic feedback can be provided to a subject and/or administrator to provide any notification associated with applicator 104 including, but not limited to, an indication to start, stop, of loss of coupling, of coupling, of power, and of duty cycle. As explained in more detail below, these indications can be understood by vibratory patterns provided to a subject and/or administrator. In another embodiment, the control circuit 200 is configured to selectively activate the vibratory device 312 to provide an indication of acoustic coupling between the applicator 104 and a body part of a subject and/or target tissue. By monitoring one or more electrical properties of the device (e.g., impedance), operative coupling between the applicator 104 and the subject can be detected. In one exemplary embodiment, the applicator includes a capacitance sensor (as described in reference to the applicator 3100 of FIGS. 27 and 28) for detecting coupling between the applicator and the subject. Suitably, the control circuit 200 can be configured to control power to the vibratory device 312 so that haptic or tactile vibration is adjusted as the strength of the coupling between the applicator 104 and the subject changes. For example, the control circuit 200 may be configured to increase tactile vibration energy as a function of the detected strength of coupling between the applicator 104 and the subject.

In one embodiment, the applicator 104 includes a level indicator that provides visual notification of a component of a transmission from applicator 104. For example, in one embodiment, the level indicator provides an indication of a level of intensity of the vibration provided by applicator 104, device 312, and/or motor 313. In some embodiments, the level indicator is a display device such as those described above. Additionally, in one embodiment, applicator 104 includes a vibratory adjustment component configured to vary the intensity, frequency, and waveform of the vibrations emitted by applicator 104, device 312, and/or motor 313. In some embodiments, the vibratory adjustment component is a button that enables a user to select a desired vibratory level that can be noted by the level indicator. Alternatively, the vibratory adjustment component can be any component that enables increasing and/or decreasing components within applicator 104 including, but not limited to, a slider, capacitive sensor, piezo resistive sensor, or user interface 108. Increases in haptic or tactile vibration may occur gradually or in stepwise fashion as the coupling strength increases to one or more predefined thresholds. Still other ways of varying tactile vibratory energy to provide an indication of coupling may also be used in other embodiments.

In addition to providing tactile feedback, the tactile vibrations provided by applicator 104, device 312, and/or motor 313, the tactile vibrations can be utilized as a vibratory massager. As noted above, vibration intensity, frequency, and waveform could be adjusted by the user to enable soft tissue massaging while also applying ultrasound therapy.

The waveform of vibration could be adjusted so user feels a pulse of vibration vs a continuous vibration. The intensity of the initiation of the vibration could be adjusted by using an amplitude modulated signal vs a square wave pulse. The amplitude-modulated signal would have a softer start of vibration because the intensity would be ramped up. The square wave would have an higher intensity when pulsed high

In another embodiment, the control circuit 200 is configured to provide energy to the vibratory device 312 to vary tactile vibratory energy as a function of the ultrasonic energy being generated by the device. For example, in devices that include a mechanism for dynamically adjusting the ultrasonic energy being provided, the control circuit 200 could adjust the energy supplied to the vibratory device 312 to vary tactile vibratory energy as a function of the ultrasonic energy being generated. Such increase may occur gradually or in stepwise fashion as the ultrasonic energy level increases to one or more predefined thresholds.

In another embodiment, the control circuit 200 is configured to control the amount of energy provided to the vibratory device 312 to vary tactile vibratory energy in such a way as to provide other indications to a subject and/or treatment administrator. For example, changes in vibratory energy may be used to provide an indication that treatment at one site has been completed and that the applicator 104 should be moved to another treatment site. This control scheme may be time-based or based on some other measurement of the amount of ultrasonic energy provided to the treatment site (e.g., total power, etc.). The vibratory device 312 may also be used to provide a tactile or haptic indication of an error of the device. In addition, haptic vibration may be used as another treatment modality in combination with ultrasound.

In one or more embodiments, the amount of tactile vibration energy that is supplied is related to a grip force of a user holding the applicator 104. For example, a force sensor, such as a load cell or piezoelectric force sensor (not shown) may be operatively connected to a grip portion of the applicator 104. When the user grips the grip portion of the applicator, the force sensor registers the gripping force and generates a signal supplied to the control circuit 200. The control circuit adjusts the duty cycle or voltage supplied to the vibratory device 312 as a function of gripping force. The adjustments to duty cycle may occur gradually or in stepwise fashion as the gripping force changes to one or more predefined thresholds. It is contemplated that adjustments to vibration energy based on gripping force may be suitable for use when tactile or haptic vibration is used as a secondary treatment modality, thus providing the user control of the level of vibration used in treatment by mere adjustment in grip strength. In addition to adjusting the duty cycle or voltage supplied to the vibratory device 312, the gripping force can be utilized to adjust any other setting of the applicator including, but not limited to, power.

Although the illustrated vibratory device 312 is used in combination with a therapeutic ultrasound device, it will be understood that other vibratory devices may be used in other ways in other embodiments. For example, a vibratory device could be used in combination with a surgical hand piece that delivers ultrasound or other energy, industrial ultrasonic welding, ultrasonic non-destructive testing devices, and/or diagnostic or imaging systems.

As explained above, the applicator 104 also includes an imaging transducer 311, which can be an ultrasound transducer. In one or more embodiments, the imaging transducer 311 may be used to determine a depth of a treatment site by, for example, identifying the depth of infected tissue or a blocked duct below the skin of the subject. After determining the depth of the treatment site, the control circuit 200 may selectively apply therapeutic ultrasound to the desired depth. For example, in one or more embodiments, the control circuit 200 may select an ultrasound frequency that is suitable for treating tissue at the desired depth. In one embodiment, the applicator may include an array of ultrasound transducers 310, each having a different resonant frequency. The control circuit 200 can be configured to select the transducer(s) 310 in the array having a resonant frequency suitable for treating target tissue at the desired depth. In other embodiments, the ultrasound device could have multiple applicators 104 for generating ultrasound at different resonant frequencies.

In one or more embodiments, the ultrasonic transducer 310 can be mounted at an angle relative to the contact surface between the applicator 104 and the subject to reduce the depth of penetration of ultrasound. Suitably, the transducer 310 may be movably (e.g., pivotably) mounted on the applicator 104 to selectively adjust the angle of the transducer relative to the contact surface to adjust a depth of treatment. In an alternative embodiment, the transducer includes one or more wedge adaptors that enable transmission at varying angles including, but not limited to any angle less than the critical angle.

As shown in FIG. 35, in some embodiments, ultrasound transducers 8310 can be mounted in an array on an adhesive patch or other patient securement structure. In one or more embodiments, the control circuit 8200 can be configured to selectively activate the transducers 8310 within the array to mimic the effect of circular movement of an applicator around a portion of a subject's skin. It is contemplated that a similar array of ultrasonic transducers 8310 allowing for simulated circular movement could be installed on a mechanical massaging device to combine ultrasonic treatment with massage. In another embodiment, one or more ultrasonic transducers could be mounted on a rotary device that provides mechanical rotation of the transducers about a treatment site.

As explained above in reference to Fig. 38, therapeutic ultrasound may be used for treatment of mastitis in one or more embodiments. In certain human applications, one or more ultrasound transducers can be incorporated into a bra for placement over a breast of a subject. Likewise, ultrasound transducers incorporated into an adhesive patch may be mounted on the breast of a subject for treatment of mastitis. Suitably, an array of transducers may be placed over a large portion of an effective area (e.g., over a substantial portion of the cup of a bra) and low power ultrasound (e.g., about 110 mW/cm$^2$, such as from about 10 mW/cm$^2$ to about 1 W/cm$^2$) may be applied for a long treatment duration. In addition, a more targeted array may be placed over a small, local treatment site and higher power ultrasound (e.g., from about 1 W/cm$^2$ to about 3 W/cm$^2$) may be applied for a shorter treatment time (e.g., from about 15 minutes to about 1 hour).

In one or more embodiments, therapeutic ultrasound may be used as an alternative to surgical castration for neutering companion animals. For example, an array of ultrasound transducers can be operatively mounted on a cup, patch, or other support article for mounting the transducers over the testes of the animal. Substantial ultrasound exposure is required to be effective. In order to avoid overheating, the transducers in the array may be alternatively activated in a high power mode to prevent localized heat buildup. Additionally or in the alternative, the ultrasound may be applied continuously over a large treatment area at low power levels for a long treatment period.

In one or more embodiments, the applicator 104 shown in FIG. 3 may be used to treat face mites, Rosacea, or other skin issues. Suitably, ultrasound energy may be applied to a treatment site at from about 0.1 W/cm$^2$ to about 3 W/cm$^2$ and from about 1 MHz to about 10 MHz. In skin treatments, ultrasound coupling gel may be combined with a chemical therapeutic to enhance effectiveness. The chemical therapeutic may be effective to disrupt the life cycle of the mites and reduce the overall mite content in the tissue.

Referring again to Fig. 33, in certain embodiments, the ultrasound bath 7100 can be used for hair conditioning. Suitably, the ultrasound applicator 7104 may include a plurality of transducers arrayed around the walls of the bath to provide ultrasound to the bath fluid. The bath fluid may comprise moisturizers, oils, vitamins, etc. for conditioning the hair of a subject. The subject's hair is placed in the bath and the transducers provide ultrasound (e.g., ultrasound of from about 25k to 10MHz (with best performance 25k to 50kHz) and from about 0.1 W/cm$^2$ to about 3 W/cm$^2$) for a treatment duration of, for example, from about 5 minutes to about 15 minutes. The ultrasound opens the hair cuticles to increase absorption of conditioning agents. After ultrasound treatment is complete, the hair can be removed from the bath, dried to damp, and leave-in conditioner may be applied.

Device 100 is also configured to enable the user to adjust the vibration frequency of the waveform to increase comfort.

In one more embodiment, the thermal properties of the therapeutic ultrasound would be used to heat an area of tissue to slow the growth of mesophilic bacteria. Mesophilic bacteria has the optimal growth temperature between 37-40C, after 40C the mesophilic bacteria growth drops dramatically as is shown below.

| Common Bacteria Found in Rhino sinusitis Patients | Temperature of Peak Bacteria Growth (°C) | Environmental Class |
|---|---|---|
| Staphylococcus aureus | 37 to 40 | Mesophile |
| Clostridium Difficile | 37 | Mesophile |
| Streptococcus pneumoniae | 37 | Mesophile |
| Haemophilus influenzae | 35-37 | Mesophile |
| Moraxella catarrhalis | 33-37 | Mesophile |
| Streptococcus pyogenes | 37 | Mesophile |

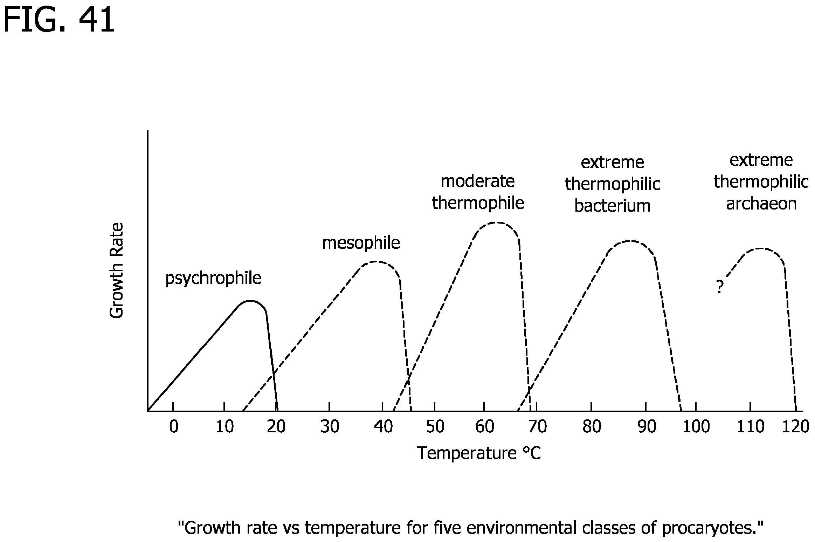

FIG. 41 illustrates the growth rate versus temperature for five environmental classes of procaryotes which includes mesophilic bacteria. To increase the thermal index of the treatment site, ultrasound is applied over infected area. During ultrasound treatment thermal index calculations are used to ensure tissue area is heated to specific a specific temperature (e.g., 38-55C). This concept could be applied over surgical sites before and after surgery to disrupt and/or reduce growth of bacteria.

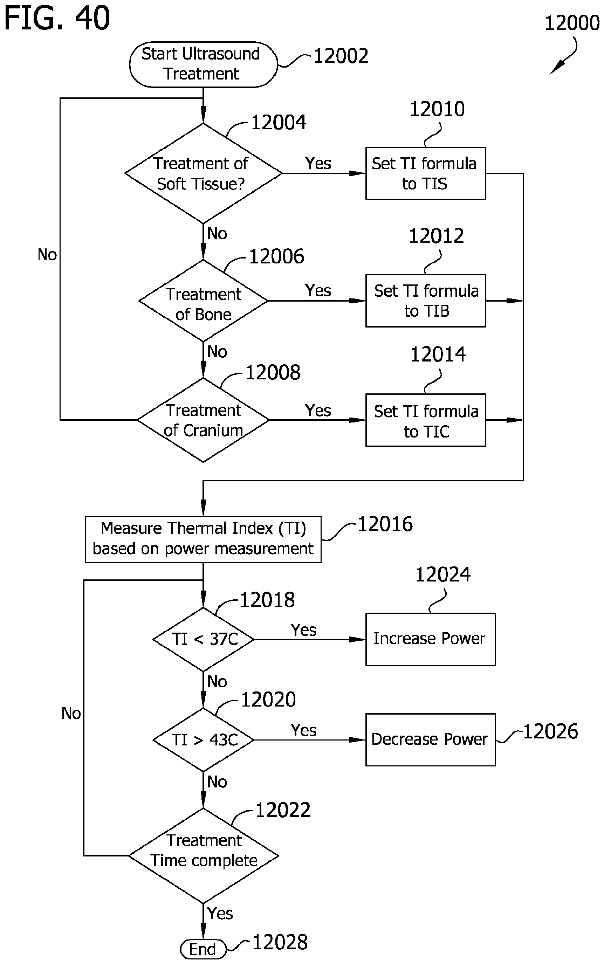

FIG. 40 is an exemplary flowchart of a method 12000 for determining the thermal index of a treatment site. To start treatment using device 100, shown in FIG. 1, the user will be required to select treatment site (soft tissue, bone or cranium), such as by touching a graphical representation of the treatment site on a display. The selection is received at steps 12004, 12006, and/or 12008. In the exemplary embodiment, a treatment site is selected through user interface 108 and transmitted to processor 208. It should be noted that the treatment site can be anywhere on or in the body. Once the treatment selection is received, 12004, 12006 and/or 12008, a thermal index formula will be set based on treatment location. In one embodiment, if soft tissue treatment site is selected in step 120004, the thermal index formula will be set with the TIS formula 12010 shown below.

$$TIS = \frac{W_0 F_{awf}}{210mW * MHz}$$

Alternatively, if a bone treatment site is selected 12006, the thermal index formula will be set with the TIB formula 12012 shown below.

$$TIB = \min[\frac{\sqrt{W_{.3} I_{spta.3}}}{50\ mW\ cm^{-1}} * \frac{W_{.3}}{4.4\ mW}]$$

In another embodiment, if a cranium treatment site is selected 12008, the thermal index formula will be set with the TIB formula 12014 shown below.

$$TIC = \frac{W_0/D_{eq}}{40 \ mW \ cm^{-1}}$$

Alternative thermal index formulas can be used for this method and are not limited to the formulas described above. Once the thermal formula is set according to treatment site, the thermal index is measured 120016. Power adjustments will be made based on 12018 and 12020. If the thermal measurement is less than 37°C as determined in 12018, then the power will be increased 12024 to increase thermal index at the treatment site. If the thermal measurement is greater than 43°C 12020, then the power level will be decreased 12026 to lower the thermal index at the treatment site. Alternative thermal rages for steps 12018 and 12020 can be used ranging between 38°C and 55°C and treatment time will be monitored 12022. If treatment time is not complete, steps 12018 and 12020 will be continually monitored. Once treatment is determined complete 12022, ultrasound therapy on the treatment site will stop 12028.

It can be seen, therefore, that high frequency ultrasound and other forms of acoustic energy can be administered to a patient alone or in combination with other treatments for therapeutic effect. High frequency ultrasound, in particular, is thought to provide any of several therapeutic benefits, depending on the mode in which it is applied. For example, the ultrasound energy can promote the breakdown of infectious biofilm, the release of enzymes, vasodilation, increased vascular or cellular permeability, the release of intracellular material, functional stimulation of certain tissues and organs, and tissue growth, among other beneficial effects. Moreover, an ultrasound transmitter can be adjusted within the context of a single treatment application to operate in various modalities that perform various functions. Thus, the systems and treatment methodologies described herein can address various clinical concerns through application of ultrasound.

The embodiments described herein may utilize executable instructions embodied in a non-transitory computer readable medium, including, without limitation, a storage device or a memory area of a computing device. Such instructions, when executed by one or more processors, cause the processor(s) to perform at least a portion of the methods described herein. As used herein, a "storage device" or "memory" is a tangible article, such as a hard drive, a solid state memory device, and/or an optical disk that is operable to store data.

Although specific features of various embodiments of the invention may be shown in some drawings and not in others, this is for convenience only. In accordance with the principles of the invention, any feature of a drawing may be referenced and/or claimed in combination with any feature of any other drawing.

This written description uses examples to disclose various embodiments, which include the best mode, to enable any person skilled in the art to practice those embodiments, including making and using any devices or systems and performing any incorporated methods.

**Claims**

1. An ultrasound treatment device comprising:

   a hand-held treatment applicator configured to acoustically interface with a treatment site of a subject;
   an ultrasound transducer disposed in the applicator, the ultrasound transducer configured to generate ultrasound energy, wherein the ultrasound transducer is coupled to the applicator to deliver the generated ultrasound energy to the treatment site when the hand-held treatment applicator is acoustically interfaced with the treatment site;
   a vibrator mounted on the applicator, the vibrator configured to generate vibratory energy, wherein the vibrator is coupled to the applicator to deliver the generated vibratory energy to the hand of the user to provide haptic feedback to the user; and
   a control circuit electrically connected to the ultrasound transducer and the vibrator, the control circuit configured to selectively supply electrical energy to the ultrasound transducer to generate ultrasound energy and to selectively supply electrical energy to the vibrator to provide haptic feedback to the user.

2. The ultrasound treatment device set forth in claim 1, further comprising a coupling sensor operatively connected to the control circuit, wherein the coupling sensor is configured to generate a coupling signal indicative of the ability to efficiently transmit ultrasonic energy from the hand-held treatment applicator into the treatment site.

3. The ultrasound treatment device set forth in claim 2, wherein the ultrasonic transducer electrical properties act as the coupling sensor.

4. The ultrasound treatment device set forth in claim 2, wherein the control circuit is configured to:

receive the coupling signal from the coupling sensor;
analyze the coupling signal to determine the ability to efficiently transmit ultrasonic energy from the hand-held treatment applicator into the treatment site; and
selectively activate the vibrator based on the determined ability to efficiently transmit ultrasonic energy from the hand-held treatment applicator.

5. The ultrasound treatment device set forth in claim 4, wherein the control circuit is configured to compare the determined ability to efficiently transmit ultrasonic energy generated from the hand-held treatment applicator to a predetermined threshold of transducer electrical properties.

6. The ultrasound treatment device set forth in claim 5, wherein the control circuit is configured to activate the vibrator if the determined ability to efficiently transmit ultrasonic energy from the hand-held treatment applicator is great than or greater than or equal to the predetermined threshold.

7. The ultrasound treatment device set forth in claim 4, wherein the control circuit is configured to selectively adjust the electrical energy supplied to the vibrator to adjust the vibratory energy generated by the vibrator based on the determined ability to efficiently transmit ultrasonic energy from the hand-held treatment applicator.

8. The ultrasound treatment device set forth in claim 7, wherein the control circuit is configured to selectively increase the amount of electrical energy supplied to the vibrator to increase the vibratory energy generated by the vibrator if the ability to efficiently transmit ultrasonic energy from the hand-held treatment applicator increases during treatment.

9. The ultrasound treatment device set forth in claim 7, wherein the control circuit is configured to selectively decrease the amount of electrical energy supplied to the vibrator to decrease the vibratory energy generated by the vibrator if the ability to efficiently transmit ultrasonic energy from the hand-held treatment applicator decreases during treatment.

10. The ultrasound treatment device set forth in claim 1, further comprising a user interface operatively connected to the control circuit, wherein the user interface is configured to allow a user to control the amount of electrical energy supplied to the vibrator by the control circuit.

11. The ultrasound treatment device set forth in claim 10, wherein the user interface comprises a grip sensor configured to generate a grip signal indicative of the amount of grip force applied to the applicator by the user.

12. The ultrasound treatment device set forth in claim 9, wherein the control circuit is configured to receive the grip signal generated by the grip sensor and adjust the amount of electrical energy supplied to the vibrator based on the received grip signal.

13. The ultrasound treatment device set forth in claim 1, wherein the control circuit is configured to selectively adjust the electrical energy supplied to the vibrator to adjust the vibratory energy generated by the vibrator.

14. The ultrasound treatment device set forth in claim 13, wherein the control circuit is configured to generate a plurality of electrical waveforms supplied to the vibrator to generate a plurality of haptic vibrations, wherein the haptic vibrations are distinguishable by the user for communicating with the user.

15. The ultrasound treatment device set forth in claim 1, wherein the control circuit is configured to operate the vibrator independent of the ultrasound transducer to provide treatment independent of the ultrasound transducer.

# FIG. 1

# FIG. 2

FIG. 3

EP 3 278 839 A1

# FIG. 4

# FIG. 5

# FIG. 6

600

FIG. 7

640

Control          Treated

642

644

# FIG. 8

660

Control          Treated

662

664

FIG. 9

702 — Power On

704 — Perform Self-test

706 — Output Calibration

708 — Tune System

710 — Coupled to Skin? — No

Yes

Start Treatment — 712

720 — Error ← Yes — Time Out? — 714

No

722 — Treatment Complete ← Yes — Treatment Complete? — 716

No

Store Data — 724

718 — Coupled to Skin? — No — Reset Treatment Time — 719

Yes

Shutdown — 722

700

# FIG. 10

800

```
                            ┌──────────────┐
                            │   Receive    │
                            │Treatment Site│────802
                            │ Information  │
                            └──────┬───────┘
                                   │
                                   ▼
              ┌──────────────┐
              │   Transmit   │
          ───►│ Image Signal │────804
              └──────┬───────┘
                     │
                     ▼
              ┌──────────────┐
              │   Receive    │
              │ Transmitted  │────806
              │   Signals    │
              └──────┬───────┘
                     │
                     ▼
              ┌──────────────┐
              │   Process    │
              │   Signals    │────808
              └──────┬───────┘
                     │
                     ▼
                    ╱╲
                   ╱  ╲
                  ╱ Is ╲
   ┌───────┐ No  ╱Applica╲
   │ Alert │◄────╱ tor Over╲────810
   └───┬───┘     ╲Treatment╱
       │          ╲ Site? ╱
      812          ╲    ╱
                    ╲  ╱
                     ╲╱
                      │ Yes
                      ▼
              ┌──────────────┐
              │    Alert     │────814
              └──────┬───────┘
                     │
                     ▼
              ┌──────────────┐
              │Enable Output │
              └──────────────┘────816
```

FIG. 11

# FIG. 12

$V_{DRIVE}$

$V_{CT}$

TO DRIVE SIGNAL

TO DRIVE SIGNAL

# FIG. 13

# FIG. 14

High Frequency
Power Amplifier

DWM
Signal

R_FILTER

C_FILTER

L_MATCH

Load

$R_4$

$R_3$

# FIG. 15

# FIG. 16

900

# FIG. 17

# FIG. 18

# FIG. 19

# FIG. 20

# FIG. 21

1004

104

# FIG. 22

1006

104

# FIG. 23

302'

313

306'

330'

310'

321

ϕ1.00

314'

320'

EP 3 278 839 A1

FIG. 24

EP 3 278 839 A1

# FIG. 25

FIG. 26

# FIG. 27

3010

TREATMENT

3100

EP 3 278 839 A1

FIG. 28

FIG. 29

EP 3 278 839 A1

## FIG. 30

# FIG. 31

# FIG. 32

# FIG. 33

7104

7101

7100

## FIG. 34

# FIG. 35

## FIG. 36

8104'

8310'

8310'

# FIG. 37

# FIG. 38

10310

10104

Cow Udder

10100

# FIG. 39

11102

11310

11100

# FIG. 40

12000

```
        ┌─────────────────┐
        │ Start Ultrasound│  ⌐12002
        │    Treatment    │
        └─────────────────┘
                 │
                 ▼
              ⌐12004                    12010
            ╱╲                      ┌──────────────┐
     ┌────╱    ╲     Yes            │ Set TI formula│
     │   ╱Treatment╲────────────────│   to TIS     │
     │   ╲of Soft  ╱                └──────────────┘
     │    ╲Tissue?╱
     │     ╲    ╱
     │      ╲╱ No
     │       │
     │       ▼  ⌐12006                 12012
  No │      ╱╲                     ┌──────────────┐
     │     ╱  ╲       Yes          │ Set TI formula│
     │    ╱Treatment╲──────────────│   to TIB     │──►
     │    ╲  of Bone ╱             └──────────────┘
     │     ╲        ╱
     │      ╲      ╱ No
     │       ╲╱ │
     │          ▼  ⌐12008              12014
     │        ╱╲                   ┌──────────────┐
     │       ╱  ╲      Yes         │ Set TI formula│
     └──────╱Treatment╲────────────│   to TIC     │──►
            ╲of Cranium╱           └──────────────┘
             ╲        ╱
              ╲      ╱
               ╲╱
                │
                ▼
   ┌─────────────────────────────┐
   │ Measure Thermal Index (TI)  │ ⌐12016
   │ based on power measurement  │
   └─────────────────────────────┘
                │
                ▼  ⌐12018               12024
             ╱╲                    ┌──────────────┐
      ┌────╱    ╲    Yes           │Increase Power│
      │    ╲TI < 37C╱──────────────│              │
      │     ╲    ╱                 └──────────────┘
      │      ╲╱ No
      │       │
      │       ▼  ⌐12020
   No │      ╱╲                    ┌──────────────┐
      │     ╱  ╲     Yes           │Decrease Power│ ⌐12026
      │     ╲TI > 43C╱─────────────│              │
      │      ╲    ╱                └──────────────┘
      │       ╲╱ No
      │        │
      │        ▼  ⌐12022
      │      ╱╲
      │     ╱Treatment╲
      └─────╲  Time   ╱
            ╲complete ╱
             ╲      ╱
              ╲╱ Yes
               │
               ▼
            ( End )  ⌐12028
```

# FIG. 41

"Growth rate vs temperature for five environmental classes of procaryotes."

Europäisches
Patentamt

European
Patent Office

Office européen
des brevets

**EUROPEAN SEARCH REPORT**

Application Number

EP 17 18 3821

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/106118 A1 (SONITEC LLC [US]) 16 July 2015 (2015-07-16) | 1-10, 13-15 | INV. A61N7/00 |
| Y | * paragraphs [0031], [0032], [0037], [0039], [0041], [0044], [0056] - [0058], [0062]; figures 1-25 * | 11,12 | ADD. A61B18/00 A61B17/00 |
| X | WO 2014/210065 A1 (ZETROZ INC [US]) 31 December 2014 (2014-12-31) * paragraphs [0028] - [0035], [0068]; figures 1-15 * | 1,2,4-6, 10 | A61B90/00 |
| Y | US 2012/116364 A1 (HOUSER KEVIN L [US] ET AL) 10 May 2012 (2012-05-10) * paragraphs [0033], [0035] - [0037], [0045], [0047], [0049] - [0051]; figures 1-14 * | 11,12 | |
| X | US 2012/330194 A1 (BRITVA ALEXANDER [IL] ET AL) 27 December 2012 (2012-12-27) * paragraphs [0063], [0064], [0068], [0073], [0090], [0093]; figures 1-8 * | 1,2,4-6, 10 | |

TECHNICAL FIELDS SEARCHED (IPC)

A61N
A61B

The present search report has been drawn up for all claims

| Place of search | Date of completion of the search | Examiner |
|---|---|---|
| The Hague | 23 November 2017 | Viidebaum, Mikk |

## ANNEX TO THE EUROPEAN SEARCH REPORT
## ON EUROPEAN PATENT APPLICATION NO.

EP 17 18 3821

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2017

| Patent document cited in search report | | Publication date | Patent family member(s) | | Publication date |
|---|---|---|---|---|---|
| WO 2015106118 | A1 | 16-07-2015 | CA | 2936453 A1 | 16-07-2015 |
| | | | US | 2016151646 A1 | 02-06-2016 |
| | | | WO | 2015106118 A1 | 16-07-2015 |
| WO 2014210065 | A1 | 31-12-2014 | EP | 3013420 A1 | 04-05-2016 |
| | | | US | 2016136462 A1 | 19-05-2016 |
| | | | WO | 2014210065 A1 | 31-12-2014 |
| US 2012116364 | A1 | 10-05-2012 | AU | 2011323174 A1 | 30-05-2013 |
| | | | AU | 2011323176 A1 | 30-05-2013 |
| | | | AU | 2011323178 A1 | 30-05-2013 |
| | | | AU | 2011323181 A1 | 30-05-2013 |
| | | | AU | 2011323183 A1 | 30-05-2013 |
| | | | AU | 2011323186 A1 | 30-05-2013 |
| | | | AU | 2011323193 A1 | 30-05-2013 |
| | | | AU | 2011323276 A1 | 23-05-2013 |
| | | | AU | 2011323279 A1 | 23-05-2013 |
| | | | AU | 2011323281 A1 | 23-05-2013 |
| | | | AU | 2011323282 A1 | 30-05-2013 |
| | | | AU | 2011323284 A1 | 30-05-2013 |
| | | | AU | 2011323286 A1 | 23-05-2013 |
| | | | AU | 2011323287 A1 | 23-05-2013 |
| | | | AU | 2011338893 A1 | 23-05-2013 |
| | | | CA | 2816853 A1 | 10-05-2012 |
| | | | CA | 2816875 A1 | 10-05-2012 |
| | | | CA | 2816877 A1 | 10-05-2012 |
| | | | CA | 2816885 A1 | 10-05-2012 |
| | | | CA | 2816888 A1 | 10-05-2012 |
| | | | CA | 2816890 A1 | 14-06-2012 |
| | | | CA | 2816899 A1 | 10-05-2012 |
| | | | CA | 2816901 A1 | 10-05-2012 |
| | | | CA | 2816908 A1 | 10-05-2012 |
| | | | CA | 2816979 A1 | 10-05-2012 |
| | | | CA | 2816980 A1 | 10-05-2012 |
| | | | CA | 2816981 A1 | 10-05-2012 |
| | | | CA | 2816982 A1 | 10-05-2012 |
| | | | CA | 2816985 A1 | 10-05-2012 |
| | | | CA | 2816986 A1 | 10-05-2012 |
| | | | CN | 103260537 A | 21-08-2013 |
| | | | CN | 103281981 A | 04-09-2013 |
| | | | CN | 103281982 A | 04-09-2013 |
| | | | CN | 103298419 A | 11-09-2013 |
| | | | CN | 103298424 A | 11-09-2013 |
| | | | CN | 103313670 A | 18-09-2013 |
| | | | CN | 103313672 A | 18-09-2013 |
| | | | CN | 103379871 A | 30-10-2013 |

EPO FORM P0459

For more details about this annex : see Official Journal of the European Patent Office, No. 12/82

page 1 of 4

## ANNEX TO THE EUROPEAN SEARCH REPORT
## ON EUROPEAN PATENT APPLICATION NO.

EP 17 18 3821

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2017

| Patent document cited in search report | Publication date | Patent family member(s) | | Publication date |
|---|---|---|---|---|
| | | CN | 103391753 A | 13-11-2013 |
| | | CN | 103391754 A | 13-11-2013 |
| | | CN | 103442658 A | 11-12-2013 |
| | | CN | 103561664 A | 05-02-2014 |
| | | CN | 103561672 A | 05-02-2014 |
| | | CN | 103648423 A | 19-03-2014 |
| | | CN | 103796600 A | 14-05-2014 |
| | | EP | 2635194 A2 | 11-09-2013 |
| | | EP | 2635203 A1 | 11-09-2013 |
| | | EP | 2635205 A1 | 11-09-2013 |
| | | EP | 2635206 A1 | 11-09-2013 |
| | | EP | 2635207 A2 | 11-09-2013 |
| | | EP | 2635214 A1 | 11-09-2013 |
| | | EP | 2635215 A1 | 11-09-2013 |
| | | EP | 2635216 A2 | 11-09-2013 |
| | | EP | 2635217 A1 | 11-09-2013 |
| | | EP | 2635219 A1 | 11-09-2013 |
| | | EP | 2635220 A1 | 11-09-2013 |
| | | EP | 2635221 A1 | 11-09-2013 |
| | | EP | 2635222 A1 | 11-09-2013 |
| | | EP | 2635223 A2 | 11-09-2013 |
| | | EP | 2635229 A1 | 11-09-2013 |
| | | JP | 5931081 B2 | 08-06-2016 |
| | | JP | 5951622 B2 | 13-07-2016 |
| | | JP | 5972892 B2 | 17-08-2016 |
| | | JP | 5992425 B2 | 14-09-2016 |
| | | JP | 6054298 B2 | 27-12-2016 |
| | | JP | 6129741 B2 | 17-05-2017 |
| | | JP | 6129742 B2 | 17-05-2017 |
| | | JP | 6138691 B2 | 31-05-2017 |
| | | JP | 2013544144 A | 12-12-2013 |
| | | JP | 2013545531 A | 26-12-2013 |
| | | JP | 2013545532 A | 26-12-2013 |
| | | JP | 2013545533 A | 26-12-2013 |
| | | JP | 2013545534 A | 26-12-2013 |
| | | JP | 2013545535 A | 26-12-2013 |
| | | JP | 2013545536 A | 26-12-2013 |
| | | JP | 2013545538 A | 26-12-2013 |
| | | JP | 2014500058 A | 09-01-2014 |
| | | JP | 2014500059 A | 09-01-2014 |
| | | JP | 2014500060 A | 09-01-2014 |
| | | JP | 2014500061 A | 09-01-2014 |
| | | JP | 2014500062 A | 09-01-2014 |
| | | JP | 2014503233 A | 13-02-2014 |
| | | JP | 2014504895 A | 27-02-2014 |
| | | US | 2012110810 A1 | 10-05-2012 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.** EP 17 18 3821

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2017

| Patent document cited in search report | Publication date | Patent family member(s) | Publication date |
|---|---|---|---|
| | | US 2012110824 A1 | 10-05-2012 |
| | | US 2012111591 A1 | 10-05-2012 |
| | | US 2012112687 A1 | 10-05-2012 |
| | | US 2012112690 A1 | 10-05-2012 |
| | | US 2012115005 A1 | 10-05-2012 |
| | | US 2012115007 A1 | 10-05-2012 |
| | | US 2012116260 A1 | 10-05-2012 |
| | | US 2012116261 A1 | 10-05-2012 |
| | | US 2012116262 A1 | 10-05-2012 |
| | | US 2012116263 A1 | 10-05-2012 |
| | | US 2012116264 A1 | 10-05-2012 |
| | | US 2012116266 A1 | 10-05-2012 |
| | | US 2012116267 A1 | 10-05-2012 |
| | | US 2012116363 A1 | 10-05-2012 |
| | | US 2012116364 A1 | 10-05-2012 |
| | | US 2012116365 A1 | 10-05-2012 |
| | | US 2012116366 A1 | 10-05-2012 |
| | | US 2012116367 A1 | 10-05-2012 |
| | | US 2012116379 A1 | 10-05-2012 |
| | | US 2012116380 A1 | 10-05-2012 |
| | | US 2012116381 A1 | 10-05-2012 |
| | | US 2012116388 A1 | 10-05-2012 |
| | | US 2012116389 A1 | 10-05-2012 |
| | | US 2012116390 A1 | 10-05-2012 |
| | | US 2012116394 A1 | 10-05-2012 |
| | | US 2012116395 A1 | 10-05-2012 |
| | | US 2012116396 A1 | 10-05-2012 |
| | | US 2012116433 A1 | 10-05-2012 |
| | | US 2015305763 A1 | 29-10-2015 |
| | | US 2016121143 A1 | 05-05-2016 |
| | | US 2016206900 A1 | 21-07-2016 |
| | | US 2016329614 A1 | 10-11-2016 |
| | | US 2016338760 A1 | 24-11-2016 |
| | | US 2017042569 A1 | 16-02-2017 |
| | | WO 2012061635 A1 | 10-05-2012 |
| | | WO 2012061638 A1 | 10-05-2012 |
| | | WO 2012061640 A1 | 10-05-2012 |
| | | WO 2012061641 A2 | 10-05-2012 |
| | | WO 2012061643 A1 | 10-05-2012 |
| | | WO 2012061645 A1 | 10-05-2012 |
| | | WO 2012061646 A1 | 10-05-2012 |
| | | WO 2012061718 A1 | 10-05-2012 |
| | | WO 2012061720 A1 | 10-05-2012 |
| | | WO 2012061722 A2 | 10-05-2012 |
| | | WO 2012061725 A1 | 10-05-2012 |
| | | WO 2012061727 A2 | 10-05-2012 |

EPO FORM P0459

For more details about this annex : see Official Journal of the European Patent Office, No. 12/82

page 3 of 4

## ANNEX TO THE EUROPEAN SEARCH REPORT
## ON EUROPEAN PATENT APPLICATION NO.

EP 17 18 3821

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2017

| Patent document cited in search report | Publication date | Patent family member(s) | Publication date |
|---|---|---|---|
| | | WO 2012061730 A1 | 10-05-2012 |
| | | WO 2012061737 A2 | 10-05-2012 |
| | | WO 2012078271 A1 | 14-06-2012 |
| US 2012330194 A1 | 27-12-2012 | NONE | |

EPO FORM P0459

For more details about this annex : see Official Journal of the European Patent Office, No. 12/82

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7299805 B

- US 6842108 B, Peter M. Bonutti